# EUROPEAN PATENT APPLICATION

(11) **EP 4 470 543 A1**
(43) Date of publication of application: **04.12.2024**
(21) Application number: 23746966.3
(22) Date of filing: 25.01.2023
(51) Int. Cl.: A61K 31/7105, A61K 9/107, A61K 9/51, A61K 39/00, A61K 39/39, A61K 48/00, A61M 5/307, A61P 37/02, C12N 15/88

(54) **RNA-CONTAINING COMPOSITION FOR TRANSDERMAL ADMINISTRATION, AND METHOD FOR ADMINISTERING SAID COMPOSITION**

(30) Priority: 25.01.2022 JP 2022009172
(71) Applicant: Kawasaki Institute of Industrial Promotion, Kawasaki-shi, Kanagawa 212-0013 (JP); NANO MRNA Co., Ltd., Tokyo 105-6226 (JP); Educational Foundation Kyorin Gakuen, Mitaka-shi, Tokyo 181-8611 (JP); KYOTO PREFECTURAL PUBLIC UNIVERSITY CORPORATION, Kyoto-shi, Kyoto 602-8566 (JP)
(72) Inventor: KATAOKA Kazunori, Kawasaki-shi, Kanagawa 212-0013 (JP); UCHIDA Satoshi, Kawasaki-shi, Kanagawa 212-0013 (JP); ABBASI Saed Amjad Yousef, Kawasaki-shi, Kanagawa 212-0013 (JP); MASAI Miki, Tokyo 105-6226 (JP); HAYASHI Akimasa, Mitaka-shi, Tokyo 181-8611 (JP)
(74) Representative: Klöckner, Christoph
(86) International application number: PCT/JP2023/002184
(87) International publication number: WO 2023/145755

(57) **Abstract**

The present invention provides a composition for transdermal administration comprising a nucleic acid. Specifically, the present invention provides a composition for transdermal administration comprising a nucleic acid, the nucleic acid comprising RNA, wherein the composition is administered by an administration method comprising spraying the composition to the surface of a target tissue, and the spraying of the composition is performed by pressurization of the composition, whereby the composition is sprayed toward the target tissue and penetrates the surface of the target tissue.

## Description

### Technical Field:

The present invention relates to a composition for transdermal administration comprising RNA and an administration method for the composition.

### Background Art:

Needleless transdermal administration devices that function by explosive charges have been proposed (Patent Literatures 1 to 7 and Non Patent Literatures 1 to 3). According to the disclosure, these devices transdermally administer proteins or DNA to subjects.

mRNA vaccines have been developed and have shown marked effectiveness for coronavirus infection. The mRNA vaccines are intramuscularly injected in the form of pseudouridine-containing mRNA encapsulated in lipid nanoparticles to subjects (Patent Literatures 8 and 9).

DNA vaccines can be inexpensively synthesized and are highly stable, and their application as vaccines is therefore expected. Unlike RNA, the DNA vaccines are excellent in, for example, long-term stability, and have the advantages of being preservable for a long period of 5 years or longer and being preservable at -20°C, for example. Furthermore, DNA can be produced much more inexpensively than RNA. Accordingly, a technique of administering an antigen-encoding vector containing DNA operably linked to a promoter using a needleless syringe has been developed (Patent Literature 10). However, the technique of Patent Literature 3 has not been confirmed to have effectiveness in clinical trials in humans and will therefore be shifted to tests at high doses according to the report (Non Patent Literature 4). Thus, as for the previous vaccine preparations, approaches of intramuscularly injecting mRNA vaccines encapsulated in lipid nanoparticles are highly effective, whereas approaches of intradermally administering DNA vaccines have not been confirmed to have effectiveness.

### Citation List:

### Patent Literature

Patent Literature 1: WO2001/078810
Patent Literature 2: Japanese Patent Laid-Open No. 2012-061269
Patent Literature 3: Japanese Patent Laid-Open No. 2012-065920
Patent Literature 4: Japanese Patent Laid-Open No. 2012-065922
Patent Literature 5: Japanese Patent Laid-Open No. 2014-147841
Patent Literature 6: Japanese Patent Laid-Open No. 2014-176759
Patent Literature 7: Japanese Patent Laid-Open No. 2016-221411
Patent Literature 8: US8,058,069B
Patent Literature 9: US9,469,664B
Patent Literature 10: WO2021/200800A

### Non Patent Literature

Non Patent Literature 1: Pharmaceutics, Drug Delivery And Pharmaceutical Technology, Vol. 108 (7), 2415-2420, 2019
Non Patent Literature 2: AAPS PharmSciTech volume 21, Article number: 19 (2020)
Non Patent Literature 3: https://doi.org/10.1101/2021.01.13.426436
Non Patent Literature 4: "Novel Coronavirus (COVID-19) DNA Vaccine: Results of Phase 1/2 and Phase 2/3 Clinical Trials", November 5, 2021, issued by AnGes, Inc. press release (https://www.anges.co.jp/pdf_news/public/YuBbtiRGK5R9o88o4TbmUsKKHAkZmLXC.pdf)

### Summary of Invention:

The present invention provides a composition for transdermal administration comprising a nucleic acid and an administration method for the composition. According to the present invention, particularly, the nucleic acid comprises RNA. Preferably, the RNA can be naked mRNA.

The present inventors have revealed that: RNA can be delivered intradermally (particularly, into the dermis) by spraying the composition to the skin and thereby transdermally administering the composition to a skin tissue; lymph node (lymphoid follicle) containing CD11-positive dendritic cells can thereby be induced to a subcutaneous tissue; during this process, the RNA is taken up by antigen-presenting cells of the epidermis and the dermis, and the antigen-presenting cells seem to accumulate in a subcutaneous tissue and form lymph node; RNA can also be delivered into the dendritic cells; and antigen-specific immunity (antibody and T cell immunity) can be induced in a subject. The present inventors have also revealed that antigen-specific T cell immunity induced by administering mRNA encoding a model antigen in combination with an adjuvant by the administration method of the present invention is at an equivalent level to that of a commercially available mRNA vaccine.

The present invention can provide the following embodiments of the invention.
(1) A composition comprising
   a nucleic acid, the nucleic acid comprising RNA (preferably naked RNA or free RNA), wherein
   the composition is administered by an administration method (preferably transdermal administration), the administration method (preferably transdermal administration) comprising spraying the composition onto the surface of a target tissue, whereby the composition is delivered into the target tissue (preferably the cytoplasm of cells in the target tissue) by penetrating the surface of the target tissue.
(2) The composition according to (1), wherein the spraying is performed so as to introduce mRNA to an area of 70 mm² or larger (e.g., performed so as to form a bulge of 5 mm or larger in diameter in the skin immediately after administration).
(3) The composition according to (1), wherein the spraying is performed so as to introduce mRNA to an area of 200 mm² or larger (e.g., performed so as to form a bulge of 8 mm or larger in diameter in the skin immediately after administration).
(4) The composition according to any of (1) to (3), wherein the spraying is performed by pressurization of the composition.
(5) The composition according to (4), wherein the pressurization is caused by the generation of a gas from a gas-generating agent.
(6) The composition according to any of (1) to (5), wherein the nucleic acid comprises naked mRNA (preferably free mRNA) (wherein the mRNA optionally comprises pseudouridine).
(7) The composition according to any of (1) to (6), wherein the composition further comprises an adjuvant.
(8) The composition according to (7), wherein the adjuvant comprises an RIG-I ligand (e.g., double-stranded RNA) or a STING activator (e.g., cGAMP).
(9) The composition according to any of (1) to (8), wherein at least a part or all of the nucleic acids are contained in lipid nanoparticles (e.g., vesicles) or contained in polyion complexes (e.g., micelle or polymersome).
(10) The composition according to any of (1) to (9),
   (i) for use in delivering the nucleic acid to the epidermis or the dermis of a subject,
   (ii) for use in expressing the nucleic acid in the epidermis or the dermis of a subject,
   (iii) for use in inducing the formation of lymphoid follicle in a subcutaneous tissue of a subject, and/or
   (iv) for use in inducing specific immunity (capable of being systemic) against a translation product of the nucleic acid in a subject.
(11) The composition according to any of (1) to (10),
   (iv-1) for use in inducing an antibody against a translation product of the nucleic acid in a subject.
(12) The composition according to any of (1) to (11),
   (iv-2) for use in inducing T cell immunity (capable of being systemic) against a translation product of the nucleic acid in a subject.
(13) A method for administering a nucleic acid to a subject in need thereof, the nucleic acid comprising RNA, the method comprising spraying a composition comprising the nucleic acid to the surface of a target tissue of the subject, whereby the composition is sprayed toward the target tissue so that the nucleic acid in the composition is delivered into the target tissue by penetrating the surface of the target tissue.
(14) A method for inducing antigen-specific immunity in a subject in need thereof, the nucleic acid comprising mRNA encoding the antigen, the method comprising spraying a composition comprising the nucleic acid to the surface of a target tissue of the subject, whereby the composition is sprayed toward the target tissue so that the nucleic acid in the composition is delivered into the target tissue by penetrating the surface of the target tissue.
(15) The method according to (13), wherein the spraying of the composition is performed by pressurization of the composition.
(16) The method according to (14), wherein the spraying of the composition is performed by pressurization of the composition.
(17) The method according to any of (13) to (16), wherein the nucleic acid consists of naked mRNA.

(21) A composition comprising
   a nucleic acid, the nucleic acid comprising naked RNA (preferably free RNA), wherein
   the composition is administered by transdermal administration, the transdermal administration comprising spraying the composition to the surface of a target tissue, whereby the composition is delivered into the target tissue (preferably the cytoplasm of cells in the target tissue) by penetrating the surface of the target tissue.
(22) A composition comprising
   a nucleic acid, the nucleic acid comprising naked RNA (preferably free RNA), wherein
   the composition is administered by transdermal administration, the transdermal administration comprising spraying the composition to the surface of a target tissue, whereby the composition is delivered into the cytoplasm of cells in the target tissue by penetrating the surface of the target tissue.
(23) The composition according to (21), wherein the spraying is performed so as to introduce mRNA to an area of 70 mm² or larger (e.g., performed so as to form a bulge of 5 mm or larger in diameter in the skin immediately after administration).
(24) The composition according to (21), wherein the spraying is performed so as to introduce mRNA to an area of 200 mm² or larger (e.g., performed so as to form a bulge of 8 mm or larger in diameter in the skin immediately after administration).
(25) The composition according to (22), wherein the spraying is performed so as to introduce mRNA to an area of 70 mm² or larger (e.g., performed so as to form a bulge of 5 mm or larger in diameter in the skin immediately after administration).
(26) The composition according to (22), wherein the spraying is performed so as to introduce mRNA to an area of 200 mm² or larger (e.g., performed so as to form a bulge of 8 mm or larger in diameter in the skin immediately after administration).
(27) The composition according to any of (21) to (26), wherein the RNA is mRNA.
(28) An administration device comprising an RNA formulation, comprising
   (α) the administration device (preferably the administration device set forth above) having a gas generation unit comprising at least a gas-generating agent, the gas generation unit having a gas discharge port, and
   (β) the RNA formulation having a composition comprising RNA and a container where the composition is stored, the container having a pressurization port for pressurization and a spray port for spraying, wherein
   the pressurization port of the RNA formulation is linked to the gas discharge port of the administration device, and the composition comprising RNA is capable of being ejected from the spray port by a pressure of a gas generated in the gas generation unit.
(28-2) An administration device comprising an RNA formulation, comprising
   (α) the administration device having a gas generation unit comprising at least a gas-generating agent, the gas generation unit having a driving unit for driving a plunger by a pressure ascribable to a gas generated from the gas-generating agent in the gas generation unit, and
   (β) the RNA formulation having a composition comprising RNA and a container where the composition is stored, the container having a pressurization port for pressurization and a spray port for spraying, wherein
   the plunger is air-tightly and liquid-tightly inserted to the pressurization port of the RNA formulation, driven by the driving unit through gas generation in the gas generation unit, and thereby pushed into the container, whereby the composition comprising RNA is capable of being ejected from the spray port.
(29) The administration device according to (28), wherein the administration device has a body having a handle part and a gas generation unit (actuator) having a container that is capable of being connected with the body and comprises at least a gas-generating agent, the gas generation unit (actuator) having a driving unit for pushing the plunger; and the RNA formulation comprises a composition comprising RNA and a container where the composition is stored, the container having a pressurization port for pressurization and a spray port for spraying, wherein the plunger is inserted to the pressurization port, and when the plunger is pushed into the container (i.e., pressurization is caused), the composition comprising RNA is sprayed from the spray port.
(29) The administration device according to (28), wherein the ejection is performed so as to introduce mRNA to an area of 70 mm² or larger (e.g., performed so as to form a bulge of 5 mm or larger in diameter in the skin immediately after administration).
(30) The administration device according to (28), wherein the ejection is performed so as to introduce mRNA to an area of 200 mm² or larger (e.g., performed so as to form a bulge of 8 mm or larger in diameter in the skin immediately after administration).
(31) The administration device according to (28-2), wherein the ejection is performed so as to introduce mRNA to an area of 70 mm² or larger (e.g., performed so as to form a bulge of 5 mm or larger in diameter in the skin immediately after administration).
(32) The administration device according to (28-2), wherein the ejection is performed so as to introduce mRNA to an area of 200 mm² or larger (e.g., performed so as to form a bulge of 8 mm or larger in diameter in the skin immediately after administration).

(41) An RNA formulation comprising
   a composition comprising RNA and a first container where the composition is stored,
   the container having a pressurization port for pressurization and a spray port for spraying, wherein a plunger is inserted to the pressurization port and is capable of being pushed into the first container, and when the plunger is pushed into the first container from the pressurization port (i.e., pressurization is caused), the composition comprising RNA is sprayed from the spray port, whereby at least the RNA penetrates tissue surface and permeates the inside of the tissue.
(42) The RNA formulation according to (41), further comprising
   a gas generation unit (actuator), the gas generation unit having a gas generation unit (actuator) having a second container comprising at least a gas-generating agent, the gas generation unit (actuator) having a driving unit for pushing the plunger, wherein the plunger is capable of being pushed into the first container via the driving unit by a gas generated from the gas-generating agent, and when the plunger is pushed into the first container from the pressurization port (i.e., pressurization is caused), the composition comprising RNA is sprayed from the spray port, whereby at least the RNA penetrates tissue surface and permeates the inside of the tissue.
(43) The RNA formulation according to (42), further having
   a body part of an administration device,
   the body part having a handle part, a switch, a voltage generation unit electrically connected with the switch, and a plug electrically connected with the voltage generation unit, and having a holder unit that permits fixation of the gas generation unit (actuator), wherein the gas generation unit is fixed to the holder unit, and the plug of the body is electrically connected with an ignition plug of the gas generation unit (actuator), whereby the RNA formulation is configured such that a voltage is generated from the voltage generation unit by switch-on so that the ignition plug is set on fire and a gas is generated from the gas-generating agent in the second container of the gas generation unit.
(44) The RNA formulation according to (41), wherein the spraying is performed so as to introduce mRNA to an area of 70 mm² or larger (e.g., performed so as to form a bulge of 5 mm or larger in diameter in the skin immediately after administration).
(45) The RNA formulation according to (41), wherein the spraying is performed so as to introduce mRNA to an area of 200 mm² or larger (e.g., performed so as to form a bulge of 8 mm or larger in diameter in the skin immediately after administration).
(46) The RNA formulation according to (42), wherein the spraying is performed so as to introduce mRNA to an area of 70 mm² or larger (e.g., performed so as to form a bulge of 5 mm or larger in diameter in the skin immediately after administration).
(47) The RNA formulation according to (42), wherein the spraying is performed so as to introduce mRNA to an area of 200 mm² or larger (e.g., performed so as to form a bulge of 8 mm or larger in diameter in the skin immediately after administration).
(48) The RNA formulation according to (43), wherein the spraying is performed so as to introduce mRNA to an area of 70 mm² or larger (e.g., performed so as to form a bulge of 5 mm or larger in diameter in the skin immediately after administration).
(49) The RNA formulation according to (43), wherein the spraying is performed so as to introduce mRNA to an area of 200 mm² or larger (e.g., performed so as to form a bulge of 8 mm or larger in diameter in the skin immediately after administration).

(61) The RNA formulation set forth above, comprising no lipid component.
(62) The RNA formulation set forth above, comprising no polyethylene glycol component.
(63) The RNA formulation set forth above, wherein the RNA is naked RNA.
(64) The RNA formulation set forth above, wherein the RNA is RNA comprising pseudouridine (e.g., m1Ψ).
(65) The RNA formulation according to (64), wherein all uridine residues in the RNA are replaced with pseudouridine (e.g., m1Ψ).
(66) The RNA formulation according to (64), wherein the RNA is naked mRNA, and all uridine residues in the RNA are replaced with pseudouridine (e.g., m1Ψ).
(67) The RNA formulation according to (66), comprising no lipid component and/or no polyethylene glycol component.

### Brief Description of Drawings

[Figure 1] Figure 1 shows results of administering naked mRNA encoding luciferase to a Balb/C mouse by intradermal injection or the transdermal administration method of the present invention, and measuring the chemiluminescence of luciferase from the mouse after administration by IVIS.
[Figure 2A] Figure 2A shows the appearance of the skin after administration.
[Figure 2B] Figure 2B shows the appearance of the skin after administration.
[Figure 3A] Figure 3A shows results of administering naked mRNA encoding luciferase to a C57BL6J mouse by intradermal injection or the transdermal administration method of the present invention, and measuring the chemiluminescence of luciferase from the mouse after administration by IVIS, and the epidermis of the mouse after administration.
[Figure 3B] Figure 3B shows the respective distributions of luciferase expression in the body of a mouse given mRNA by the transdermal administration method of the present invention and luciferase expression in the body of a mouse given lipid nanoparticles (LNP) by intradermal administration.
[Figure 4] Figure 4 shows results of IVIS-observing chemiluminescence from the skin of a mouse to which naked mRNA encoding luciferase was administered by the transdermal administration method of the present invention.
[Figure 5] Figure 5 shows results of immunohistochemical staining of lymphoid follicles formed in skin tissues of a mouse to which naked mRNA encoding GFP was administered by the transdermal administration method of the present invention.
[Figure 6A] Figure 6A shows the induction of OVA-specific immunity in a mouse in which 4.5 µg of m1Ψ-modified mRNA encoding ovalbumin (OVA) was administered to each of right and left ventral parts.
[Figure 6B] Figure 6B shows the induction of OVA-specific immunity in a mouse in which half the indicated dose of N1m1Ψ-modified mRNA encoding ovalbumin (OVA) was administered to each of right and left ventral parts.
[Figure 7] Figure 7 shows the induction of cytotoxic T cells (CTL) in a mouse in which 4.5 µg of unmodified mRNA encoding ovalbumin (OVA) was administered to each of right and left ventral parts.
[Figure 8] Figure 8 shows the induction of IgG, IgG1, and IgG2a in a mouse in which 4.5 µg of unmodified mRNA or m1Ψ-modified mRNA encoding ovalbumin (OVA) was administered to each of right and left ventral parts in the presence or absence of an adjuvant (double-stranded RNA or cGAMP).

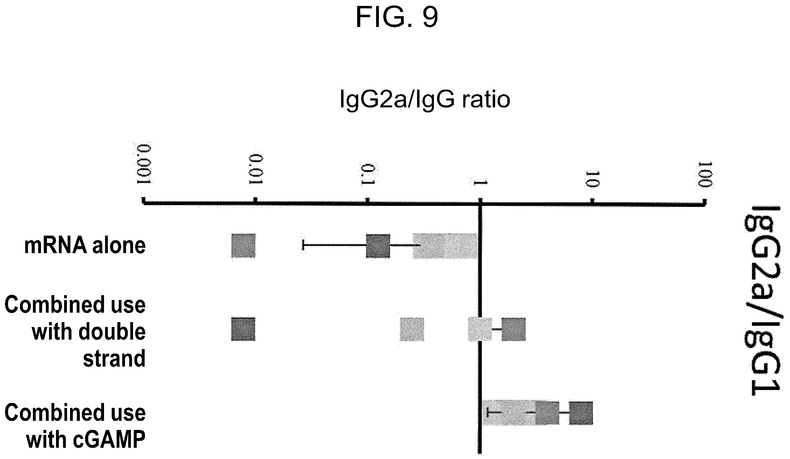

[Figure 9] Figure 9 shows an IgG2a/IgG1 ratio after m1Ψ-modified mRNA administration calculated from the results shown in Figure 8.
[Figure 10] Figure 10 shows an OVA-specific IgG level in plasma obtained from a mouse prepared by administering 9 µg of m1Ψ-modified mRNA encoding ovalbumin (OVA) to one ventral part of the mouse, administering the same dose thereas to an ipsilateral ventral part or a contralateral ventral part 3 weeks later, and raising the mouse for 2 weeks.
[Figure 11] Figure 11 shows the number of OVA-specific INF-γ-positive cells and results of ELISpot assay in splenocytes obtained from a mouse prepared by administering 4.5 µg of unmodified mRNA encoding SARS-CoV-2 helicase to right and left ventral parts twice and raising the mouse for 1 week.
[Figure 12A] Figure 12A shows the induction of cytotoxic T cells (CTL) in a mouse in which 4.5 µg of unmodified mRNA encoding ovalbumin (OVA) was administered to each of right and left ventral parts.
[Figure 12B] Figure 12B shows IVIS-observing chemiluminescence derived from luciferase in a mouse to which naked mRNA encoding luciferase was administered by the transdermal administration method of the present invention and a mouse to which an LNP formulation thereof was administered by intramuscular injection.
[Figure 13] Figure 13 shows swollenness in the liver and the spleen of a mouse administered with LNP.
[Figure 14A] Figure 14A shows an antibody titer and neutralization capacity of an S protein-specific antibody in plasma of a Balb/C mouse group to which mRNA encoding the spike protein (S protein) of coronavirus (Wuhan strain) was administered by the transdermal administration method of the present invention, and the activation of S protein-specific cellular immunity in splenocytes of the mouse group.
[Figure 14B] Figure 14B shows an antibody titer of an S protein-specific antibody in plasma of a C57BL6J mouse group to which mRNA encoding the spike protein (S protein) of coronavirus (Wuhan strain) was administered by the transdermal administration method of the present invention, and the activation of S protein-specific cellular immunity in splenocytes of the mouse group.
[Figure 14C] Figure 14C shows the expression of inflammatory cytokines (specifically, interferon β1 and interleukin 6) in the liver and the spleen of each of a mouse group administered with mRNA encoding the spike protein (S protein) of coronavirus (Wuhan strain) by the transdermal administration method of the present invention, and a group administered with mRNA encapsulated in LNP by subcutaneous administration.
[Figure 14D] Figure 14D shows an antibody titer and neutralization capacity of an S protein-specific antibody in plasma of a cynomolgus monkey to which mRNA encoding spike protein (S protein) of coronavirus (Wuhan strain) was administered by the transdermal administration method of the present invention.
[Figure 15] Figure 15 is a schematic view of the RNA formulation of the present invention.
[Figure 16] Figure 16 is a schematic view of the RNA formulation of the present invention in a form connected with an administration device.
[Figure 17] Figure 17 is a schematic view of an administration device having a gas generation unit.
[Figure 18] Figure 18 is a schematic view of a gas generation unit.
[Figure 19] Figure 19 shows a body of an administration device having a holder of a gas generation unit.
[Figure 20] Figure 20 is a schematic view of a body of an administration device before linking to a gas generation unit.

### Description of Embodiments

### <Definition of term>

In the present specification, defined terms have meanings as defined. Undefined terms have meanings generally used in the art. A singular form does not exclude a plurality. The term "comprise" means that an element other than specified matter may be included. The term "consist of" means that an element other than specified matter is not substantially included (e.g., commingling of the element inevitable in production is merely involved, the element is included in only an amount that does not substantially influence functions, or the element is included only below a detection limit) or not included.

In the present specification, the "subject" can be an animal or a plant. The animal or the plant includes an animal and a plant. Examples of the animal include vertebrates, for example, mammals and birds. Examples of the mammal include primates such as humans, rodents such as mice and rats, and livestock mammals such as bovines, horses, sheep, donkeys, sheep, goats, llamas, and camels. Examples of the birds include chickens.

In the present specification, the "target tissue" means a tissue such as the skin (preferably the epidermis and the dermis), and various organs. Thus, the surface of the target tissue includes the surface of the skin, the surface of a tissue such as mucosa, and the surface of various organs. In the present invention, a composition is administered so as to penetrate (or pass through) the surface of the target tissue. An administration format that performs administration so as to penetrate (or pass through) skin surface is referred to as transdermal administration. In the present specification, the "skin" is a layer that covers the external surface of the body. The skin is constituted by the epidermis and the dermis. The epidermis is an ectoderm-derived tissue and is composed mainly of keratinocytes. The epidermis has a basal layer containing basal cells actively dividing in contact with the dermis, on a basement membrane. Epidermal cells arising from basal cells move from the basal side to the outside while changing to prickle cells, granular cells, and keratinocytes. The dermis is a mesoderm-derived tissue situated as a layer beneath the epidermis. The dermis has collagen-producing fibroblasts and immunocytes such as mast cells involved in immune functions or inflammation. The dermis has sweat gland such as eccrine gland and apocrine gland.

In the present specification, the "nucleic acid" includes deoxyribonucleic acid (DNA), and ribonucleic acid (RNA), and their modified nucleic acids. Examples of the nucleic acid include, but are not particularly limited to, nucleic acids comprising only DNA, nucleic acids comprising only RNA, nucleic acid comprising only DNA and RNA, nucleic acids comprising DNA and modified nucleic acids, nucleic acids comprising RNA and modified nucleic acids, and nucleic acids comprising DNA, RNA, and modified nucleic acids.

In the present specification, the "messenger RNA" (mRNA) is an RNA having a region encoding a protein and can produce the protein as a translation product in cells. The mRNA usually has a 5' cap structure, a 5' untranslated region (UTR), a coding region, 3'UTR, and a polyadenine sequence. The 5' cap structure can be, for example, a cap containing N7-methylguanosine (m7G) (e.g., m7GpppG cap and 3'-O-methyl-m7GpppG cap). The 5'UTR and The 3'UTR promote, for example, the translation of the protein from the mRNA.

In the present specification, the "modified nucleic acid" is a derivative of DNA or RNA and can be DNA or RNA modified for the purpose of enhancing the ability to hybridize, stability against degradation, heat stability, or the like. Examples of the modified nucleic acid include, but are not particularly limited to, fluorescent dye-modified nucleic acids, biotinylated nucleic acids, and nucleic acids harboring a cholesteryl group. RNA may have 2'-O-methyl modification, 2'-fluoro modification, or 2'-methoxyethyl (MOE) modification of a base, or replacement of a phosphodiester bond of a nucleic acid backbone with a phosphorothioate bond, in order to enhance stability. Examples of the modified nucleic acid include bridged nucleic acids. Examples of such an artificial nucleic acid include bridged nucleic acids (BNAs) such as locked nucleic acid (LNA) which is bridged DNA in which 2' oxygen atom and 4' carbon atom are joined via a methylene bridge, ENA in which 2' oxygen atom and 4' carbon atom are joined via an ethylene bridge, BNACOC in which 2' oxygen atom and 4' carbon atom are joined via a -CH₂OCH₂- bridge, and BNANC in which 2' oxygen atom and 4' carbon atom are joined via a -NR-CH₂- {wherein R is methyl or a hydrogen atom} bridge, cMOE in which 2' oxygen atom and 4' carbon atom are joined via a -CH₂(OCH₃)- bridge, cEt in which 2' oxygen atom and 4' carbon atom are joined via a -CH₂(CH₃)- bridge, AmNA in which 2' and the 4' carbon atoms are joined via an amide bridge, scpBNA in which 2' oxygen atom and 4' carbon atom are joined via a methylene bridge and cyclopropane is formed at position 6', peptide nucleic acid (PNA) having a polymer backbone with N-(2-aminoethyl)glycine linked through an amide bond instead of deoxyribose or ribose, and morpholino oligo with bases linked through a morpholine ring (e.g., US9,469,664B which is incorporated herein by reference in its entirety). The modified nucleic acid may have acidic nature or does not have to be acidic. As for mRNA, examples thereof include mRNA comprising a modified nucleoside. Examples of the mRNA comprising a modified nucleoside include modified nucleosides described in US8,278,036B which is incorporated herein by reference in its entirety. Examples of the modified nucleoside include pseudouridine, which is known as a modified nucleoside for *in vivo* expression of mRNA. The modified nucleoside is capable of substituting an unmodified nucleoside. Examples of the pseudouridine include 1-methyl-3-(amino-5-carboxypropyl)pseudouridine (m¹acp³Ψ), 1-methylpseudouridine (m1Ψ), 2'-O-methylpseudouridine (Ψm), 5-methyldihydrouridine (m5D), and 3-methylpseudouridine (m3Ψ), each of which is capable of substituting uridine. The modified mRNA preferably comprises pseudouridine and preferably, may further comprise 5-methylcytidine.

In the present specification, the "vesicle" means a particle that can store a substance in the inside. In the present specification, the nanovesicle means a vesicle having a hydrodynamic diameter of less than 1 µm. The vesicle can be, for example, micelle that is constituted by a monolayer membrane and can store a substance in the inside, and a hollow vesicle that is constituted by a bilayer membrane and can store a substance in the inside.

In the present specification, the "lipid nanoparticle" (LNP) means a nanoparticle which is a nanovesicle constituted by a lipid molecule (e.g., micelle and liposome) or a complex of a lipid molecule and a component such as a nucleic acid (lipoplex). Examples of the lipid nanoparticle include nucleic acid-lipid particles described in US8,058,069B which is incorporated herein by reference in its entirety. Amphiphilic lipids are capable of forming lipid nanoparticles in an aqueous solution. Those skilled in the art can appropriately select a lipid and form the lipid nanoparticle from the lipid.

In the present specification, the RNA is naked RNA or is RNA encapsulated in a vesicle. In this context, the "naked RNA" means RNA present in a form that is not encapsulated in a vesicle. As such naked RNA, RNA unbound with other constituents is particularly referred to as free RNA.

In the present specification, the "isolation" refers to removal of at least one or more impurities contained in a system. The isolation means, for example, taking a substance of interest out of a cell. In the present specification, the "purification" means improvement in purity of a substance of interest. In a composition to be applied to the human body (e.g., a pharmaceutical composition for a human and a vaccine for a human), usually, its constituents have each been isolated or purified.

### <Composition of present invention>

The composition of the present invention comprises a nucleic acid. In a certain embodiment of the present invention, the nucleic acid comprises RNA (particularly, isolated RNA). In a certain embodiment of the present invention, the nucleic acid comprises only RNA. In a certain embodiment of the present invention, the nucleic acid comprises DNA or a modified nucleic acid and RNA. In a preferred embodiment of the present invention, the RNA is single-stranded RNA. In a certain embodiment of the present invention, the RNA can be RNA in a form encapsulated in a vesicle (e.g., a lipid nanoparticle, micelle, or liposome). In a certain embodiment of the present invention, the RNA can be in the form of a complex with a lipid molecule (lipoplex). In a certain embodiment of the present invention, the RNA can be a complex with a cationic polymer (polyion complex, for example, polyion complex micelle or polymersome). In a certain preferred embodiment of the present invention, the RNA is naked RNA. In a preferred embodiment of the present invention, the nucleic acid can be naked single-stranded RNA (particularly, free RNA).

In a preferred embodiment, the RNA can be messenger RNA (mRNA). In a certain preferred embodiment, each base of the mRNA consists of an unmodified base. In a certain preferred embodiment, the mRNA may comprise a modified nucleic acid. Particularly preferred mRNA can comprise pseudouridine instead of uridine. In more preferred mRNA, some or all uridine residues (particularly, all uridine residues) are changed to pseudouridine. In a particularly preferred embodiment, the pseudouridine is m1Ψ.

The mRNA encodes a protein. Examples of the protein encoded by the mRNA include components specific for heterogeneous organisms, for example, pathogens, or a portion thereof, and components or a portion thereof that are specific for cancer cells. Examples of the pathogen include pathogenic viruses, pathogenic microbes, and pathogenic parasites. Examples of the pathogenic virus include viruses selected from the group consisting of human immunodeficiency (HIV), hepatitis A virus, hepatitis B virus, hepatitis C virus, herpes virus, adenovirus, influenza virus, flavivirus, echovirus, rhinovirus, coxsackievirus, coronavirus (e.g., severe acute respiratory syndrome-associated coronavirus (SARS-CoV), Middle East respiratory syndrome coronavirus (MERS), and SARS-CoV-2), respiratory syncytial virus, mumps virus, rotavirus, measles virus, rubella virus, parvovirus, vaccinia virus, human T cell leukemia (HTL) virus, dengue virus, papillomavirus, molluscum virus, poliovirus, rabies virus, John Cunningham (JC) virus, and arbovirus encephalitis virus. Examples of the pathogenic microbe include pathogenic bacteria. Examples of the pathogenic bacterium include bacteria selected from the group consisting of chlamydia, bacteria of the order *Rickettsiales,* mycobacteria, staphylococci, streptococci, pneumococcus, meningococcus, gonococcus, the genus *Klebsiella,* the genus *Proteus,* the genus *Serratia,* the genus *Pseudomonas,* the genus *Legionella,* diphtheria, the genus *Salmonella,* rods, and bacteria of cholera, tetanus, botulism, anthrax, plague, leptospirosis, and Lyme disease. Examples of the pathogenic microbe include pathogenic fungi. Examples of the pathogenic fungus include pathogenic fungi selected from the group consisting of *Candida* (*albicans, krusei, glabrata, tropicalis,* etc.), *Cryptococcus neoformans, Aspergillus* (*fumigatus, niger,* etc.), the order *Mucorales* (the genus *Mucor,* the genus *Absidia,* and the genus *Rhizopus*)*, Sporothrix schenckii, Blastomyces dermatitidis, Paracoccidioides brasiliensis, Coccidioides immitis,* and *Histoplasma capsulatum.* Examples of the pathogenic parasite include pathogenic parasites selected from the group consisting of *Entamoeba histolytica, Balantidium coli, Naegleria fowleri,* the genus *Acanthamoeba, Giardia lamblia,* the genus *Cryptosporidium, Pneumocystis carinii, Plasmodium vivax, Babesia microti, Trypanosoma brucei, Trypanosoma cruzi, Leishmania donovani, Toxoplasma gondii,* and *Ancylostoma braziliense.* Thus, in a certain embodiment of the present invention, the mRNA can encode a constituent (preferably a surface antigen) of such a pathogen or a portion thereof. Among the surface antigens of the pathogens, particularly, an antigen that can be utilized by a pathogen for the infection of cells, for example, S protein of beta coronavirus, can be preferably encoded by the mRNA of the present invention. Examples of the cancer include cancers selected from the group consisting of urinary bladder cancer, breast cancer, uterine cancer, endometrial cancer, ovary cancer, colorectal cancer, colon cancer, head and neck cancer, lung cancer, stomach cancer, germ cell cancer, bone cancer, squamous cell cancer, skin cancer, central nervous system neoplasm, lymphoma, leukemia, sarcoma, virus-related cancer, small-cell lung cancer, non-small cell lung cancer, gastrointestinal cancer, Hodgkin's or non-Hodgkin's lymphoma, pancreatic cancer, glioblastoma, glioma, uterine cervical cancer, ovary cancer, liver cancer, myeloma, salivary gland cancer, kidney cancer, basal cell cancer, melanoma, prostate cancer, vulvar cancer, thyroid cancer, testicle cancer, esophageal cancer, and head and neck cancer. In a certain embodiment of the present invention, the mRNA can be an antigen specific for such a cancer (cancer-specific antigen, preferably cancer-specific surface antigen) or a portion thereof. The cancer-specific antigen can be, for example, a neoantigen or a portion characteristic of the neoantigen. Examples of the cancer-specific antigen also include cancer testis antigens (e.g., proteins of the MAGE family and NY-ESO-1), differentiation antigens (e.g., melanoma tyrosine kinase, Melan-A/MART-1, and prostate cancer PSA), overexpressed cancer antigens (e.g., Her-2/Neu, survivin, telomerase, and WT-1), cancer-inducing mutants of β-catenin, cancer-inducing mutants of CDK4, MUC-1, particularly, MUC-1 having an altered glycosylation pattern, and human papilloma virus E6 or E7. In this respect, those skilled in the art can appropriately select an antigen according to the type of an infection or a cancer in a subject and the type of the antigen expressed in the cancer, and prepare the mRNA suitable for the present invention.

In a certain preferred embodiment, the composition of the present invention is substantially free of or free of a lipid nanoparticle and a constituent thereof. In a certain embodiment, the composition of the present invention is substantially free of or free of a polyethylene glycol component. In a certain embodiment, in the composition of the present invention, the nucleic acid comprises RNA, and the RNA consists of naked RNA (preferably mRNA). In a certain embodiment, in the composition of the present invention, the nucleic acid comprises RNA, and the RNA comprises RNA (preferably mRNA) in a form encapsulated in a lipid nanoparticle.

The composition of the present invention may further comprise an adjuvant. The adjuvant is not particularly limited and can be selected from the group consisting of, for example, poly-I:C polynucleotide, double-stranded RNA, lipopolysaccharide (LPS), CD40 ligand, a ligand of Toll-like receptor TLR1, TLR2, TLR3, TLR4, TLR5, TLR6, TLR7, TLR8, TLR9 or TLR10, a ligand of TLR13, a ligand of NOD-like receptor, retinoic acid-inducible gene I (RIG-I) ligand, an immunostimulatory nucleic acid, immunostimulatory RNA (isRNA), CpG-DNA, a STING activator, for example, cGAMP (e.g., natural cGAMP or, for example, cyclic GMP-AMP, particularly, one intracellularly induced through cyclic GMP-AMP (cGAS), for example, 2',3'-cGAMP), an incomplete Freund's adjuvant, and an aluminum-based adjuvant (e.g., alum such as aluminum hydroxide and aluminum phosphate) (see WO2017/083963 which is incorporated herein by reference in its entirety).

In a certain preferred embodiment, the double-stranded RNA may hybridize with mRNA through the use of its partial nucleotide sequence (see WO2018/124181 which is incorporated herein by reference in its entirety). In this embodiment, the RNA may have a blunt 5' end, and/or the blunt 5' end may have triphosphate.

The composition of the present invention may further have a pharmaceutically acceptable carrier, additive, or excipient. The composition of the present invention can be, for example, an aqueous preparation and may further comprise a pharmaceutically acceptable salt (e.g., sodium salt), pH buffer, and/or metal chelating agent. The composition of the present invention is RNase-free and has been sterilized (e.g., sterilized by electron beam irradiation).

The composition of the present invention can be, for example, an aqueous solution, a cream, an ointment, an emulsion (e.g., an O/W emulsion), a powder, granules, or a gel. However, the composition of the present invention is in a dosage form suitable for spraying by pressurization.

### <Administration method for composition of present invention>

The composition of the present invention is administered by the administration method of the present invention. Specifically, the administration method of the present invention comprises spraying the composition (e.g., an aqueous solution) to the surface of a target tissue. The spraying is performed with intensity sufficient or suitable for delivering the composition into the target tissue by penetrating the surface of the target tissue. Thus, the administration method of the present invention comprises spraying the composition to the surface of a target tissue, with intensity sufficient for delivering the composition into the target tissue by penetrating the surface of the target tissue. The sufficient intensity can be, for example, intensity that allows the composition to reach a depth on the order of 0.2 mm to 3 mm from the surface of a target tissue.

In a certain preferred embodiment, the spraying can be performed by pressurization of the composition. The pressurization can be performed by pressurization of the composition by a gas. In a certain preferred embodiment, the composition is stored in a container, the container having a pressurization port for pressurization inside the container and a spray port for spraying, and the spraying can be performed from the spray port. Then, a gas is generated or expanded and thereby ejected into the container containing the composition so that the inside of the container can be pressurized. By this pressure, the composition can be sprayed from the spray port provided on the container. The gas for use in pressurization can be generated from, for example, a gas-generating agent. The gas-generating agent is capable of generating the gas by combustion. The generation of the gas from the gas-generating agent can be caused directly or via the combustion of an igniting charge. The generated gas reaches the pressurization port and pressurizes the inside of the container. By contrast, the spray port has a relatively low pressure, and the composition is sprayed through the spray port from the pressurized inside of the container. In a certain preferred embodiment, the pressurization exploits explosive power ascribable to gas generation. The explosive power can be controlled explosive power. In a certain preferred embodiment, as mentioned later, the spraying can be performed by an RNA formulation 10 given below.

The igniting charge is not particularly limited and can be an igniting charge selected from the group consisting of, for example, any one gunpowder of a gunpowder containing zirconium and potassium perchlorate, a gunpowder containing titanium hydride and potassium perchlorate, a gunpowder containing titanium and potassium perchlorate, a gunpowder containing aluminum and potassium perchlorate, a gunpowder containing aluminum and bismuth oxide, a gunpowder containing aluminum and molybdenum oxide, a gunpowder containing aluminum and copper oxide, and a gunpowder containing aluminum and iron oxide, and combinations of two or more thereof. For example, a gunpowder zirconium-potassium perchlorate (ZPP) mixture can be used as the igniting charge. For example, a gunpowder containing nitrocellulose, diphenylamine, and potassium sulfate can be used as the gas-generating agent. Any of various gas-generating agents for use in gas generators for airbags or gas generators for seatbelt pretensioners can also be used as the gas-generating agent. The gas-generating agent can be preferably a smokeless gunpowder containing 95 to 99% by weight (e.g., 98%) of nitrocellulose, diphenylamine (0.5 to 1.5% by weight, for example, 0.8% by weight), and potassium sulfate (0.7 to 2% by weight, for example, 1.2% by weight). The igniting charge preferably generates no gas at a level that causes substantial variation in pressure during its combustion. The gas-generating agent is capable of generating a pressure of 5 to 50 MPa, preferably 20 to 40 MPa, more preferably 25 to 34 MPa, during gas generation. Those skilled in the art should be able to appropriately select and use a gas-generating agent and an igniting charge. The pressure can be maximized in, for example, approximately 10 to 30 msec after gas generation.

The spraying may be performed a plurality of times by pressurization at the same or different pressures. In this respect, for example, the following approach can be effective: the first pressurization enhances skin permeability, and the second pressurization allows a drug solution to permeate the skin.

In the present invention, the composition can be allowed to widely permeate the skin by transdermal administration through a jet spray. For example, the skin having an area equal to or larger than a circle of 2 mm in diameter (approximately 3.14 mm² in terms of an area) receives the composition (particularly, mRNA, preferably mRNA encoding an immunogen). Thus, a bulge equal to or larger than a circle of 2 mm in diameter can be formed in the skin surface by transdermal administration through a jet spray. In a certain embodiment, the skin having an area corresponding to the area of a circle of 2 mm or larger in diameter, 3 mm or larger in diameter, 4 mm or larger in diameter, 5 mm or larger in diameter, 6 mm or larger in diameter, 7 mm or larger in diameter, 8 mm or larger in diameter, 9 mm or larger in diameter, or 10 mm or larger in diameter receives the composition (particularly, mRNA, preferably mRNA encoding an immunogen). In a certain embodiment, the skin having an area of approximately 3.14 mm² or larger, 10 mm² or larger, 20 mm² or larger, 30 mm² or larger, 40 mm² or larger, 50 mm² or larger, 60 mm² or larger, 70 mm² or larger, 80 mm² or larger, 90 mm² or larger, 100 mm² or larger, 110 mm² or larger, 120 mm² or larger, 130 mm² or larger, 140 mm² or larger, 150 mm² or larger, 160 mm² or larger, 170 mm² or larger, 180 mm² or larger, 190 mm² or larger, or 200 mm² or larger receives the composition (particularly, mRNA, preferably mRNA encoding an immunogen). In these embodiments, a bulge can be formed in the skin having such an area. In a certain embodiment, the mRNA encodes a beneficial protein to be expressed in endogenous immunocytes of the skin. In a certain embodiment, the mRNA encodes an immunogen. In a certain embodiment, the mRNA encodes a viral antigen. In a certain embodiment, the mRNA encodes a cancer antigen.

### <Medical application of composition of present invention>

The composition of the present invention can be used in medical application. The composition of the present invention can introduce, for example, a nucleic acid comprising RNA to a target tissue. When the RNA is functional RNA, an intended medical effect can thereby be obtained. When the RNA encodes a protein, the RNA enables the protein to be expressed in a target tissue.

The composition of the present invention can be used, for example, in delivering a nucleic acid to a skin tissue (e.g., the epidermis or the dermis) of a subject. The composition of the present invention can be preferably used in delivering a nucleic acid to the dermis of a skin tissue of a subject. The composition of the present invention can be used in expressing a nucleic acid in the dermis of a subject.

The composition of the present invention can be used in forming lymph node (lymphoid follicle) in a subcutaneous tissue of a subject. The lymphoid follicle can contain, for example, immunocytes such as dendritic cells. The composition of the present invention can be used in delivering a nucleic acid to and/or expressing a nucleic acid in lymph node (lymphoid follicle) of a subcutaneous tissue.

The composition of the present invention can also be used, for example, in inducing specific immunity against a translation product of the nucleic acid (i.e., a protein or the like encoded by the nucleic acid) contained in the composition in a subject. The specific immunity is preferably systemic. The composition of the present invention can also be preferably used in inducing an antibody against a translation product of the nucleic acid in a subject. The antibody can be, for example, IgG. Further, the composition of the present invention can be preferably used in inducing T cell immunity against a translation product of the nucleic acid in a subject. The T cell immunity can be the induction of CD4 single-positive T cells and/or the induction of CD8 single-positive T cells.

Thus, the composition of the present invention can be used as a vaccine, preferably a vaccine for an infection or a cancer. In this embodiment, the present invention can provide the composition of the present invention that can be a vaccine, preferably a vaccine for an infection or a cancer.

### <Method for administering nucleic acid according to present invention>

The present invention provides a method for administering a nucleic acid. The nucleic acid comprises RNA. The nucleic acid used can be the nucleic acid as described in the section of the composition of the present invention, so that the description is omitted here with reference to the description in the section of the composition of the present invention.

The present invention provides a method for administering a nucleic acid to a subject in need thereof, the method comprising spraying the composition to the surface of a target tissue of the subject, whereby the composition is sprayed toward the target tissue and delivered into the target tissue by penetrating the surface of the target tissue. The target tissue can be an organ or a tissue such as a skin or mucosal tissue. Details of the spraying method are as described in the section of the administration method for the composition of the present invention, so that the description is omitted here with reference thereto.

In the method for administering a nucleic acid according to the present invention, the nucleic acid involves spraying the composition to the surface of a target tissue, whereby the nucleic acid is delivered into the target tissue by penetrating the surface of the target tissue.

In the method for administering a nucleic acid according to the present invention, the spraying can be performed using an administration device suitable therefor. The administration device can have an enclosure unit where the composition is enclosed, a pressurization unit that pressurizes the composition enclosed in the enclosure unit, and a channel unit that defines a channel such that the composition pressurized by the pressurization unit is injected to the surface of a target tissue. The pressurization unit can pressurize the composition in the pressurization unit in order to allow the composition to penetrate the surface, and an elevated pressure in the pressurization unit serves as driving force for injection. The elevated pressure in the pressurization unit results from gas release from a gas-generating agent.

### <Method for inducing immunity according to present invention>

The present invention provides a method for inducing antigen-specific immunity in a subject, the method comprising spraying the composition (comprising mRNA encoding the antigen) to skin surface of the subject, whereby the composition is sprayed toward the target tissue and delivered into the target tissue by penetrating the surface of the target tissue. Details of the composition and details of the administration method are as mentioned above. In a certain embodiment, the antigen-specific immunity can be antigen-specific humoral immunity (antibody production). In a certain embodiment, the antigen-specific immunity can be cellular immunity (T cell immunity, particularly, T cell immunity brought about by CD8 single-positive T cells and/or T cell immunity brought about by CD4 single-positive T cells). In a certain embodiment, the antigen-specific immunity can be both antigen-specific humoral immunity (antibody production) and cellular immunity (T cell immunity, particularly, T cell immunity brought about by CD8 single-positive T cells and/or T cell immunity brought about by CD4 single-positive T cells). In a certain embodiment, the method for inducing antigen-specific immunity can involve inducing lymph node (lymphoid follicle) in the skin. The lymph node (lymphoid follicle) can contain dendritic cells.

### <Other embodiments of invention>

The present invention provides use of the nucleic acid in the manufacture of the composition of the present invention. The present invention also provides use of the nucleic acid in the manufacture of the composition for use in medical application of the present invention.

In a certain embodiment, the present invention can provide an RNA formulation 10 comprising a composition 13 comprising RNA and a container 14 where the composition 13 is stored, the container 14 having a pressurization port 11 for pressurization and a spray port 12 for spraying. In a preferred embodiment, the container 14 has a cylinder shape, and a plunger 11a may be air-tightly and liquid-tightly inserted to the pressurization port 11. Specifically, pressurization may be performed by pushing the plunger 11a into the container 14. In this way, while the contact of a gas generated from a gas-generating agent with the composition is avoided, a pressure ascribable to the gas is conveyed to the container via the plunger so that the composition can be sprayed from the spray port. For this purpose, the plunger 11a can have a plunger rubber 11b at its tip. The presence of the plunger rubber 11b is advantageous in air-tightly and liquid-tightly pushing the plunger into the container 14. In a preferred embodiment, the container 14 has a cylinder shape, and the plunger 11a or the plunger rubber 11b is air-tightly and liquid-tightly movable in the cylinder, whereby the composition stored in the container 14 can be effectively sprayed from the spray port 12. The plunger rubber can be made of rubber (e.g., silicone rubber) so as to be air-tightly and liquid-tightly movable. In a certain preferred embodiment, the spray port 12 is aseptically sealed. In a certain preferred embodiment, the spray port 12 may be a hole having a bore sufficient for ejection. The bore of the hole can be appropriately adjusted in order to secure an ejection pressure of the composition in the inside. The spray port 12 may be an opening. The RNA formulation may be aseptically placed in a bag. This RNA formulation can be used in administration to a subject via tissue surface (e.g., skin surface) of the subject. The RNA can be naked RNA and can be, for example, free RNA. In a preferred embodiment, the RNA can be mRNA. The internal capacity of the container 14 can be appropriately determined. In the present specification, the container 14 is also referred to as a first container.

The RNA formulation can be administered to a subject by ejecting the composition comprising RNA inside the container from the spray port by pressurization from the pressurization port. The pressurization method and the composition comprising RNA are as mentioned above. Thus, the overlapping description about the pressurization method and the composition comprising RNA is omitted here with reference to the above description.

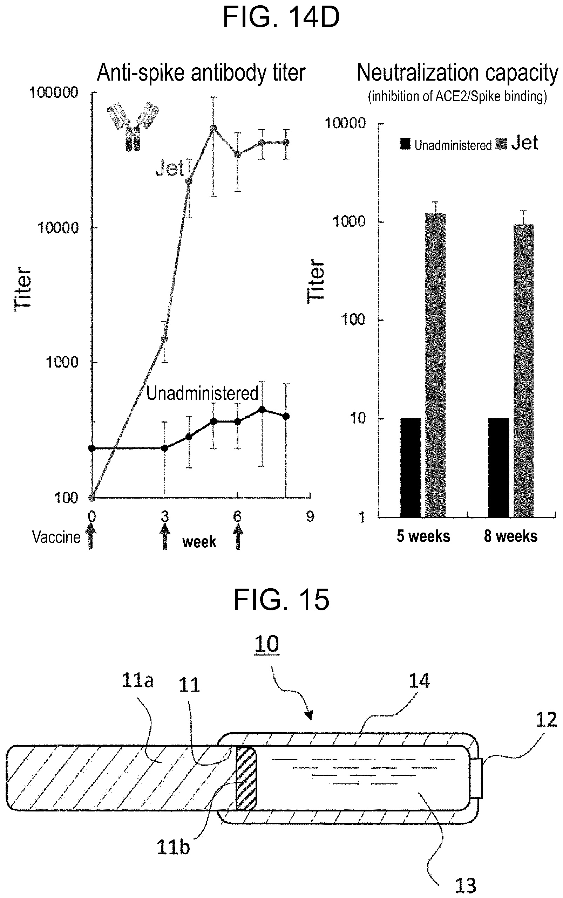

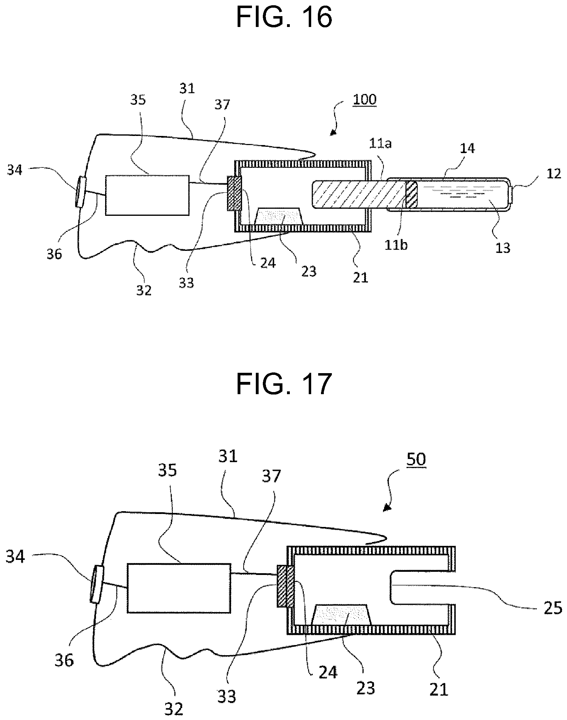

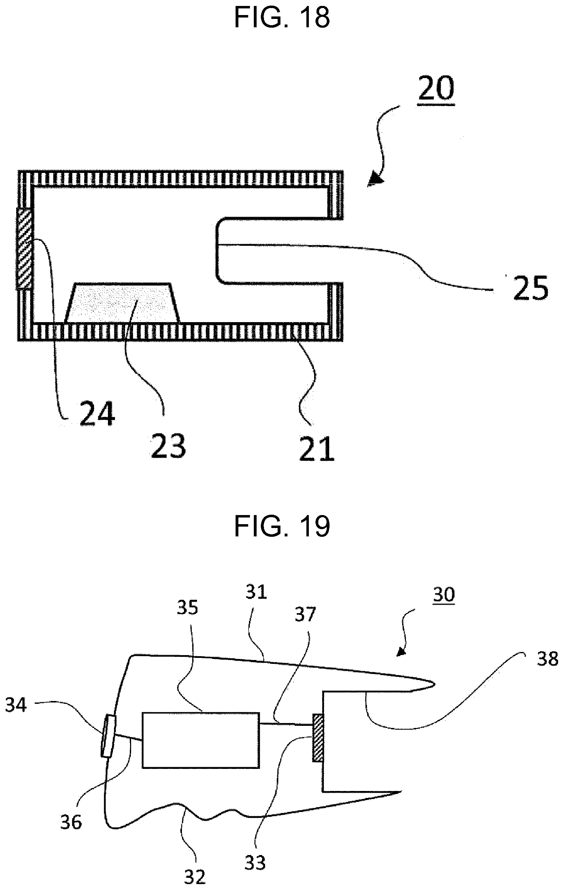

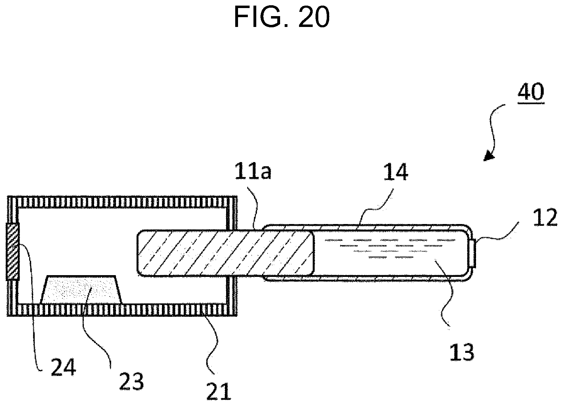

The RNA formulation can be connected with an administration device (e.g., see Figure 17), and the composition comprising RNA can be sprayed from the spray port through the use of pressurization by the administration device (e.g., see Figure 16). An administration device 50 has a body 31 having a handle part 32 and a gas generation unit (actuator) 20 having a container 21 that is capable of being connected with the body 31 and comprises at least a gas-generating agent 23, and the gas generation unit (actuator) 20 has a driving unit 25 for pushing the plunger. The driving unit 25 is connected with the plunger 11a, and the plunger 11a can be pushed into the container 14 via the driving unit 25 in association with gas generation from the gas-generating agent 23. The administration device 50 and the RNA formulation 10 are configured as separate bodies (e.g., see Figures 15 and 17) and may be separately sold and linked to each other before use, for example, or may be sold in a linked state (e.g., see Figure 16). In this way, the RNA formulation can be exchanged on a dose basis. In the administration device as well, the body 30 having the handle and the gas generation unit 20 are configured as separate bodies (e.g., see Figures 18 and 19) and may be separately sold and linked to each other before use, for example, or may be sold in a linked state (e.g., see Figure 17). In this way, the body part can be recycled by exchanging the gas generation unit in a cartridge-like manner on a dose basis. Alternatively, the RNA formulation and the gas generation unit may be integrated (see Figure 20). Thus, this embodiment provides an RNA formulation having a gas generation unit. In the RNA formulation, the driving unit 25 of the gas generation unit is configured so as to push the plunger 11a into the container 14 in association with gas generation from the gas-generating agent 23. The gas generation unit may further comprise an igniting charge. For example, the gas-generating agent 23 may further comprise an igniting charge. In the present specification, the container 21 is also referred to as a second container.

The gas generation unit (actuator) 20 may have an ignition plug 24 for generating a gas from the gas-generating agent 23. In this way, a gas can be generated by ignition or the like of the gas-generating agent 23. The body 30 may have a plug 33 capable of being electrically connected with the ignition plug 24. The plug is linked to a voltage generation unit 35. The linking can be performed via a conductive wire 37 or the like. The voltage generation unit 35 is also linked to a switch 34. The linking can be performed via a conductive wire 36 or the like. At turn-on of the switch 34, the voltage generation unit 35 can generate a voltage and send the voltage to the plug 33. The ignition plug 24 electrically connected with the plug 33 generates a gas from the gas-generating agent 23 in response to the voltage from the plug 33. The gas thus generated actuates the driving unit 25, which can in turn push the plunger 11a into the container 14. The voltage generation unit may be driven by electricity supplied from the outside or may be driven by electricity supplied from an internal power source.

In a certain embodiment, the present invention provides the RNA formulation having a gas generation unit comprising at least a gas-generating agent, wherein the gas generation unit is linked, for use to an administration device having a gas discharge port.

The RNA formulation of the present invention can be administered using (α) an administration device having a gas generation unit comprising at least a gas-generating agent, the gas generation unit having a gas discharge port. The RNA formulation of the present invention can be linked to the gas discharge port of the administration device via the pressurization port.

In a certain embodiment, the present invention provides an administration device comprising an RNA formulation, comprising
(α) the administration device (preferably the administration device described above) having a gas generation unit comprising at least a gas-generating agent, the gas generation unit having a driving unit that is driven in association with gas generation in the gas generation unit, and
(β) the RNA formulation having a composition comprising RNA and a container where the composition is stored, the container having a pressurization port for pressurization and a spray port for spraying, wherein
the pressurization port of the RNA formulation is linked to the driving unit of the administration device, and the container is capable of being pressurized by the driving unit that is driven in association with gas generation in the gas generation unit, whereby the composition comprising RNA is capable of being ejected from the spray port.

More specifically, as shown in Figure 16, the present invention can provide an RNA formulation 100 comprising a composition 13 comprising RNA and a container 14 where the composition 13 is stored, the container 14 having a pressurization port 11 for pressurization and a spray port 12 for spraying, wherein a plunger 11a is air-tightly and liquid-tightly inserted to the pressurization port 11, and pressurization is performed by pushing the plunger 11a into the container 14, whereby a pressure associated with gas generation in a gas generation unit is conveyed to the container via the plunger so that the composition is capable of being sprayed from the spray port,
the administration device further having a body part 30 of an administration device,
the body part having a handle part 32, a switch 34, a voltage generation unit 35 electrically connected with the switch 34, and a plug 33 electrically connected with the voltage generation unit 35, and having a holder unit 38 that permits fixation of a gas generation unit (actuator) 20, wherein the gas generation unit 20 is fixed to the holder unit 38, and the plug 33 of the body is electrically connected with an ignition plug 24 of the gas generation unit (actuator), whereby the RNA formulation is configured such that a voltage is generated from the voltage generation unit 35 by turn-on of the switch 34 so that the ignition plug 24 is set on fire and a gas is generated from the gas-generating agent 23 in the second container 21 of the gas generation unit.

The linking between the gas discharge port of the administration device and the pressurization port can be performed directly or indirectly. The linking between the gas discharge port of the administration device and the pressurization port can be performed through, for example, a gas feed tube that intervenes between the gas discharge port and the pressurization port.

In a certain embodiment, the RNA formulation can be a vaccine formulation.

All literatures cited herein are incorporated herein by reference in their entirety.

### Examples

### Example 1

### Material

CleanCap(TM) FLuc mRNA, EGFP mRNA, 5moU-modified Cre mRNA, ovalbumin (OVA) mRNA, N1-methylpseudouridine (m1Ψ)-modified OVA mRNA, 5' cap analog (ARCA), m1Ψ-5'-triphosphate, and m1Ψ-modified COVID-19 spike protein mRNA were purchased from Trilink Biotechnologies (San Diego, CA, USA). An intradermal administration device, Actranza(TM) (actuator: 1A1MD-5, container unit: 1C20-5) was purchased from Daicel Corp. cGAMP and OVA were purchased from Sigma. A goat anti-mouse IgG antibody conjugated with horseradish peroxidase (HRP) was purchased from R&D Systems, Inc., and IgG1 and IgG2a antibodies conjugated with HRP were purchased from Abcam plc. Anti-IFNy ELISpot was purchased from Mabtech AB. PepTivator OVA Epitope Mix was purchased from Miltenyi Biotec, and PepMix(TM) SARS-CoV-2 (Nsp13) Epitope Mix was purchased from JPT Peptide Technologies. MEGAscript(TM) and mMESSAGE mMACHINE(TM) T7 Transcription Kits were purchased from Thermo Fisher Scientific Inc.

### mRNA synthesis

A plasmid prepared by incorporating coronavirus NSP13 into a pSP73 vector containing a 120-base polyA/T strand was commissioned to GenScript for production. Then, *in vitro* transcription using mMESSAGE mMACHINE(TM) Kit and extraction using RNeasy Mini Kit (Qiagen, Hilden, Germany) were performed.

### Double-stranded mRNA synthesis

24-base RNA (GGUGUGUGUGUGUGUGUGUGUGUG: SEQ ID NO: 1) was transcribed *in vitro* from a DNA template having the sequence of TAATACGACTCACTATAGGTGTGTGTGTGTGTGTGTGTGTG (SEQ ID NO: 2) using
MEGAshortscript. This RNA was mixed with 5 types of 43-base RNAs having the following sequences and OVA mRNA and heat-treated at 65°C for 5 minutes followed by cooling to 30°C over 10 minutes for hybridization to prepare double-stranded mRNA.

### [Formula 1]

GGUUCAGGAUGUCCCGCUUCACACACACACACACACACACACC (SEQ ID NO: 3)
GGGCAGGAUGGGGUACCUUCACACACACACACACACACACACC (SEQ ID NO: 4)
GUCCUCGUCCUUGAAGGAUCACACACACACACACACACACACC (SEQ ID NO: 5)
GCUUCUCGAAGUUGAUGUACACACACACACACACACACACACC (SEQ ID NO: 6)
GGCGAUGUGCUUGAUGCUUCACACACACACACACACACACACC (SEQ ID NO: 7)

### Preparation of lipid nanoparticle (LNP)

1,2-Distearoyl-sn-glycero-3-phosphocholine (DSPC), alpha-(3'-{[1,2-di(myristyloxy)propanoxy]carbonylamino}propyl)-ω-methoxy, polyoxyethylene (PEG2000-DMG), cholesterol, and (6Z,9Z,28Z,31Z)-heptatriaconta-6,9,28,31-tetraen-19-yl-4-(dimethylamino)butanoate (D-Lin-MC3-DMA) in an ethanol solvent, and a 3-fold volume of a solution of mRNA in a citrate buffer (pH = 3) were added at a flow rate of 12 mL/min using NanoAssemblr(TM) Ignite(TM) (Precision NanoSystems Inc., Vancouver, BC, Canada). Then, the mixture was diluted 40-fold with PBS and ultrafiltered through a 30 kD filter. An mRNA encapsulation rate of 80% or more was confirmed by Ribogreen assay.

### IVIS observation

20 µL of a solution containing 1 µg of FLuc mRNA was administered to a C57BL/6J mouse or a Balb/C mouse by a method using a syringe (manual) or a method using Actranza device. IVIS measurement was performed 10 minutes after addition of 200 µL of a 5 mg/mL luciferin solution.

### Vaccine

C57BL/6J females were purchased from Charles River Laboratories Japan, Inc. and used at the age of 7 to 9 weeks in vaccine experiments. mRNA was diluted with a 10 mM HEPES buffer (pH = 7.4) and administered at 20 µL to a ventral region. For effect determination, blood was collected from the inferior vena cava, and the blood was centrifuged at 2000 × g at 4°C for 10 minutes and subjected to antibody detection. The spleen was also collected and subjected to ELISpot and *in vivo* cytotoxic T cell tests. For LNP in a test on NSP13 (helicase) mRNA, 50 µL of PBS was injected to the musculus quadriceps femoris on both sides.

### ELISpot

The spleen thus collected was dipped in 5 mL of RPMI-1640 medium (containing 10% FBS, 1 mM sodium pyruvate, 10 mM HEPES, 50 µM mercaptoethanol, and 1% penicillin-streptomycin), and a suspension of splenocytes was prepared using a metal mesh. The suspension was allowed to pass through a 40 µm nylon mesh (Cell strainer, Falcon). Manual MACS(TM) Magnetic Separator (Miltenyi Biotec) was used to separate CD4-positive and CD8-positive T cells. These cells were inoculated at a density of 2.5 × 10⁵ cells/well to anti-IFNy ELISpot 96-Well Plate. An epitope mix was added at 0.025 µg/well for OVA and at 0.2 µg/well for NSP13. The plate was cultured overnight and then stained in accordance with the protocol, and the number of spots was observed with ELISpot plate reader (AID GmbH, Germany).

### Quantification of antibody

Plasma antibody titers were quantified by enzyme linked immunosorbent assay (ELISA). For ELISA plate preparation, OVA protein was prepared at a concentration of 2 µg/mL with a 50 mM carbonate buffer (pH = 9.6), and this solution was added at 50 µL/well to Clear Flat-Bottom Immuno Nonsterile 96-Well Plates (Thermo Fisher Scientific Inc.) and cultured overnight at 4°C. The plate was washed three times with PBS containing 0.5% v/v Tween 20 (PBS-T), and 100 µL of PBS-T containing 1% BSA and 2.5 mM EDTA was added to each well, followed by culture at room temperature for 1 hour. Then, the plate from which the solution was removed was used in ELISA. A plasma sample was diluted with PBS-T containing 1% BSA and 2.5 mM EDTA, then added at 50 µL/well to the plate, and cultured overnight at 4°C. Afte washing three times with PBS-T, 50 µL of goat anti-mouse IgG-HRP (1:8000), goat anti-mouse IgG1-HRP, or IgG2a-HRP (1:10000) was added to each well, followed by culture at room temperature for 2 hours. Finally, HRP substrate was added at 100 µL/well, followed by culture at room temperature for 30 minutes. The reaction was terminated by the addition of 2 M sulfuric acid, and absorbance at 492 nm was observed with a plate reader (Tecan, Switzerland). The antibody titer was determined with absorbance of 0.1 as a threshold.

### In vivo cytotoxic T cell (CTL) test

Splenocytes treated with an OVA epitope (SIINFEKL) were administered to a vaccinated mouse, and the survival of the splenocytes in the mouse was evaluated to evaluate immunity brought about by cytotoxic T cells. In the SIINFEKL treatment, splenocytes were recovered from an unvaccinated mouse and hemolyzed with ACK lysis buffer to prepare SIINFEKL-treated cells and untreated cells. For the SIINFEKL-treated cells, the cells were treated with SIINFEKL at 37°C for 1 hour and then treated with 5 µM carboxyfluorescein diacetate (CFSE) at 37°C for 10 minutes. For the untreated cells, the cells were treated with 0.5 µM CFSE at 37°C for 10 minutes. The SlINFEKL-treated cells and the untreated cells were mixed in equal amounts and administered at 1 × 10⁷ cells/mouse to the mouse. On the next day, the spleen was recovered, and a suspension was prepared. The number of cells was evaluated in a flow cytometer (LSRFortessa(TM) Cell Analyzer, BD Biosciences). Cytotoxic activity (%) was determined according to the following formula:[(The number of SIINFEKL-untreated cells) - (The number of SIINFEKL-treated cells)] / (The number of SIINFEKL-untreated cells) × 100

### Preparation of lipoplex

For the preparation of 1,2-di-O-octadecenyl-3-trimethylammoniumpropane (DOTMA)/DOPE liposome, 200 µL of a solution of 60 mM DOTMA (Mw = 670.6 g/mol) in chloroform and 200 µL of a solution of 1,2-dioleoyl-sn-glycero-3-phosphoethanolamine (DOPE) (744 g/mol) in chloroform are mixed and dried using argon gas in a glass tube to form a film on the wall of the glass tube. 4 ml of water is added thereto, and the mixture is vigorously vortexed and sonicated at 23 Hz for 5 minutes. The final concentration of DOTMA is 3 µmol/mL. This liposome and mRNA are mixed at a nitrogen/phosphate (N/P) ratio of 0.5. The final concentration of NaCl is 150 mM.

### Preparation and observation of HE-stained specimen

An injection site was marked, and a skin tissue of 1 cm square was collected such that the injection site was positioned at the center. The skin tissue was fixed in neutral buffered formalin for 24 hours. The tissue thus fixed was cut into a width of approximately 2 mm, and paraffin was infiltrated into the cut pieces of the skin tissue using a paraffin infiltration apparatus. After embedding, tissue paraffin blocks (FFPE blocks) were prepared by cooling. The prepared paraffin blocks were sliced into 4 µm, stained with hematoxylin-eosin, and then observed under a biological microscope.

### Multiplex fluorescent staining

The FFPE blocks prepared as mentioned above were subjected to multiplex fluorescent immunohistological staining. An antigen was retrieved by the autoclave method using a citrate buffer of pH 7.0, and the samples were then incubated overnight at 4°C using a diluted antibody mixture of an anti-CD11c antibody Cell Signaling Technology, Inc., #97585, diluted 200-fold) and an anti-GFP antibody (Abcam plc, ab6673, diluted 1000-fold). The samples were washed and then incubated at room temperature for 1 hour with a diluted secondary antibody mixture (Rabbit 567 and Goat 488, Invitrogen Corp.). The samples were washed, subjected to nuclear staining with DAPI, and observed and photographed under a fluorescence microscope (Keyence Corp., BZ-X).

### Results

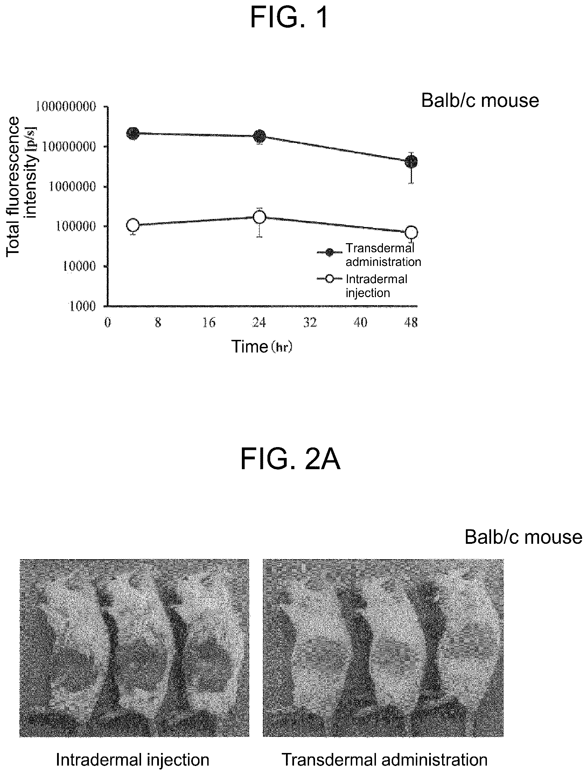

Methods for transdermally administering naked mRNA encoding luciferase were studied. The first method was intradermal injection. A group (intradermal administration group) was provided in which a solution containing 1 µg of mRNA was intradermally injected to a ventral part of each mouse (Balb/c, n = 3). Total luminescence intensity in IVIS observation was on the order of approximately 100,000 (see Figure 1).

Devices that release DNA from a nozzle and transdermally administer the DNA by a jet spray are known as devices for transdermal administration of DNA (see Japanese Patent Laid-Open Nos. 2012-061269, 2012-065920, 2012-065922, 2014-147841, 2014-176759, and 2016-221411; Pharmaceutics, Drug Delivery And Pharmaceutical Technology, Volume 108, Issue 7, p. 2415-2420, July 01, 2019; and AAPS PharmSciTech volume 21, Article number: 19 (2020), https://doi.org/10.1101/2021.01.13.426436; these literatures are incorporated herein by reference in their entirety). An administration device based on these techniques is sold as Actranza(TM) Lab by Daicel Corp. Hereinafter, the "transdermal administration" refers to transdermal administration using this administration device. Actranza(TM) Lab is not a device that administers a substance to the skin by only one gas spray, but exploits two gas sprays. Among the two gas sprays, it is considered that the first spray contributes to improvement in skin penetration, and the second spray contributes to drug permeation.

The naked mRNA encoding luciferase was transdermally administered to mice (Balb/c, n = 3) using Actranza(TM) Lab (referred to as a "transdermal administration group"). As a result, total luminescence intensity in IVIS observation exceeded approximately 10,000,000 (see Figure 1). This group was found to exhibit more than 100 times the expression efficiency of the intradermal administration group.

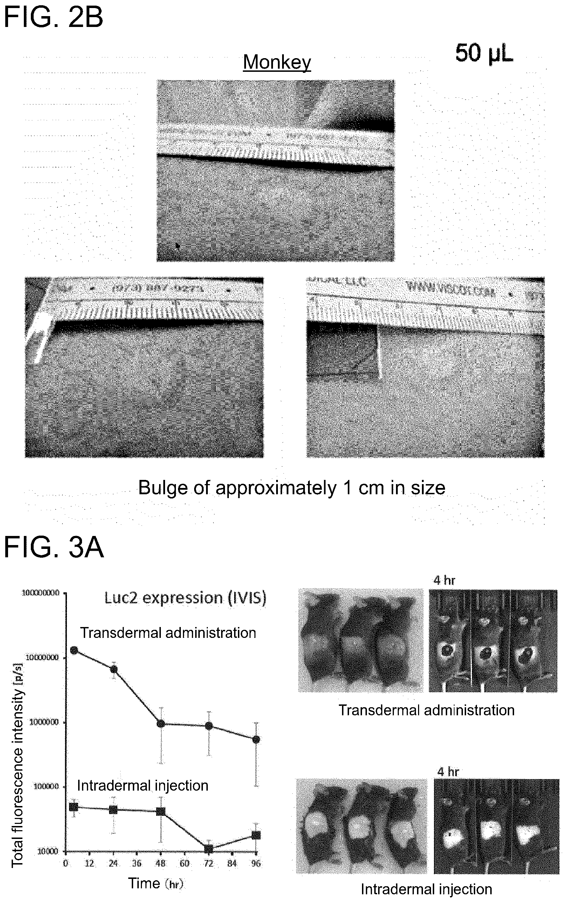

Figure 2A shows the appearance of an administration site. According to Figure 2A, the appearance of the skin did not differ between the intradermal administration group and the transdermal administration group. Next, the naked mRNA encoding luciferase (dose: 50 µL) was also transdermally administered to the shaved skin of a monkey using Actranza(TM) Lab. As a result, according to Figure 2B, administration was achieved such that the diameter of a bulge formed after administration was approximately 1 cm, suggesting that the mRNA permeated a wide area of the skin. The mRNA was able to permeate a wide area and was thus considered advantageous from the viewpoint of increasing the possibility of delivery to immunocytes (particularly, dendritic cells) scattered in the skin.

Actranza(TM) performs two jet sprays by one operation. The first spray improves skin penetration, and the second spray can allow a drug solution to widely permeate the skin (e.g., see Japanese Patent Laid-Open No. 2021-061269). The two sprays are expected to be effective for increase in the amount of a drug solution permeating the skin. Through the use of the jet sprays, Actranza(TM) can spray a drug solution to a wide region and is consequently expected to allow a drug solution to permeate a wide region of the skin.

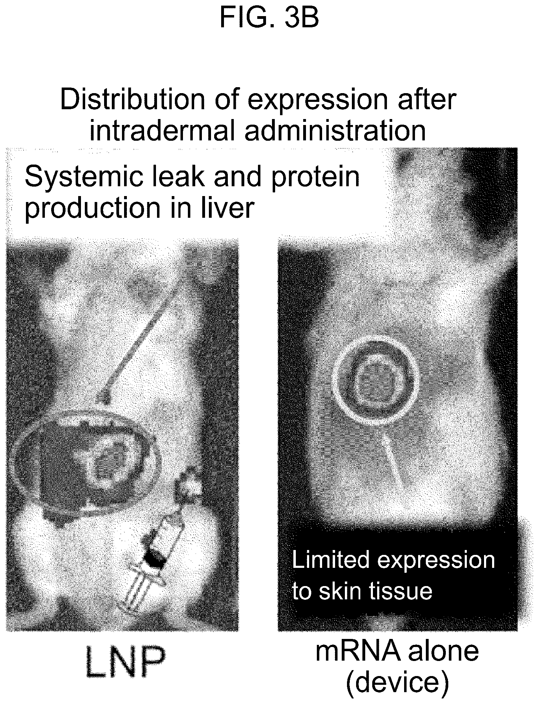

The same experiment as above was also conducted on mice (C57BL6J). The results were as shown in Figure 3A. The mice of this strain also exhibited a much larger gene expression level in the transdermal administration group than that of the intradermal administration group. A luciferase expression site after administration was observed by IVIS. As a result, as shown in Figure 3B, the gene expression site was limited to the administration site in the transdermal administration group. LNP in which the mRNA encoding luciferase was encapsulated was intradermally injected, and a luciferase expression site after injection was observed by IVIS. As a result, as shown in Figure 3B, strong expression of luciferase was found in the liver, not at the administration site.

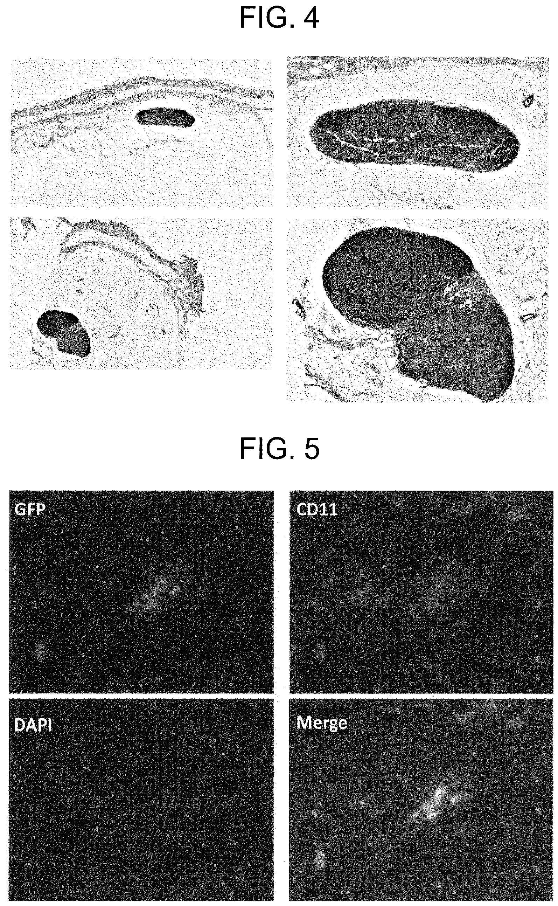

Tissue sections of the skin were prepared as to the administration site in the mice of the transdermal administration group and subjected to hematoxylin-eosin staining. In the transdermal administration group, surprisingly, the induction of lymph node (lymphoid follicle) in subcutaneous tissues was found (see Figure 4). Such induction of lymphoid follicle was not found in the intradermal administration group. This suggested that immunity was induced in the administration site in the transdermal administration group. It is usually considered that lipid nanoparticles (LNP), even if intradermally administered, for example, leak out of the skin and migrate throughout the body and a portion thereof migrates to lymph node and activates the immune system in the lymph node. Thus, a different mechanism of immune response and a different risk of developing adverse reactions from those of LNP administration are suggested.

Immunocytes in the lymphoid follicle induced as described above were observed. mRNA encoding green fluorescent protein (GFP) was prepared in the same manner as above and transdermally administered to mice in the same manner as above. 1 day later, tissues containing intradermally induced lymphoid follicle were subjected to immunohistochemical staining. Staining using an anti-CD11 antibody was performed in order to detect dendritic cells. Whether the introduced mRNA was expressed in dendritic cells was confirmed. As a result, as shown in Figure 5, the fluorescent signal of GFP was colocalized with a signal from CD11. This revealed that mRNA was taken up by dendritic cells in the induced lymphoid follicle and expressed in the dendritic cells. In this Example, the mRNA was administered to the subjects with intensity that allowed the mRNA to reach the dermis but not reach a subcutaneous tissue. Hence, the results described above suggest that dendritic cells of the epidermis or the dermis, after mRNA uptake, accumulated in subcutaneous tissues and formed lymph node.

### Example 2: Induction of antigen-specific immunity by transdermal administration

m1Ψ-modified naked mRNA encoding ovalbumin was prepared as described in Example 1. 4.5 µg of the mRNA and 4.5 µg of the mRNA per mouse were transdermally administered to the right and left ventral parts, respectively, using Actranza(TM) Lab (transdermal administration group) or intradermally injected thereto (intradermal administration group). On week 3, the same amount as above of the naked mRNA encoding ovalbumin was further administered for boost immunization in the same manner as above. On week 5, plasma was recovered and subjected to ELISA assay, while splenocytes were recovered and subjected to ELISpot assay.

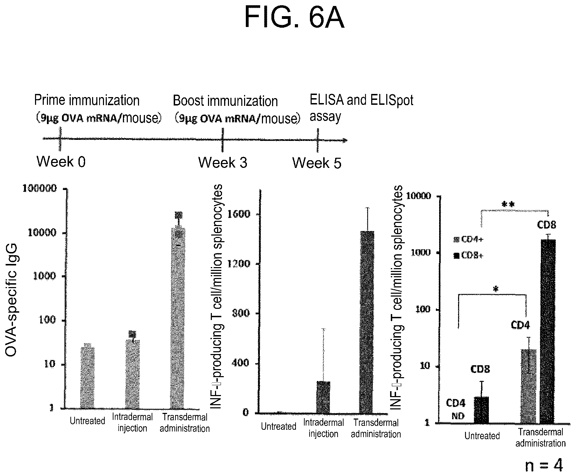

The results were as shown in Figure 6A. The induction of OVA-specific IgG by intradermal administration exhibited no significant difference from that in the untreated group, whereas marked induction of OVA-specific IgG was found for the transdermal administration (see Figure 6A, left). As a result of ELISpot assay, the number of spots of INF-γ-producing T cells was markedly increased in the transdermal administration group compared with intradermal injection (see Figure 6A, middle). Among the T cells, CD4 single-positive T cells and CD8 single-positive T cells were separately subjected to ELISpot assay, the results of which revealed that both the CD4 single-positive T cells and the CD8 single-positive T cells were significantly induced by transdermal administration (see Figure 6A, right).

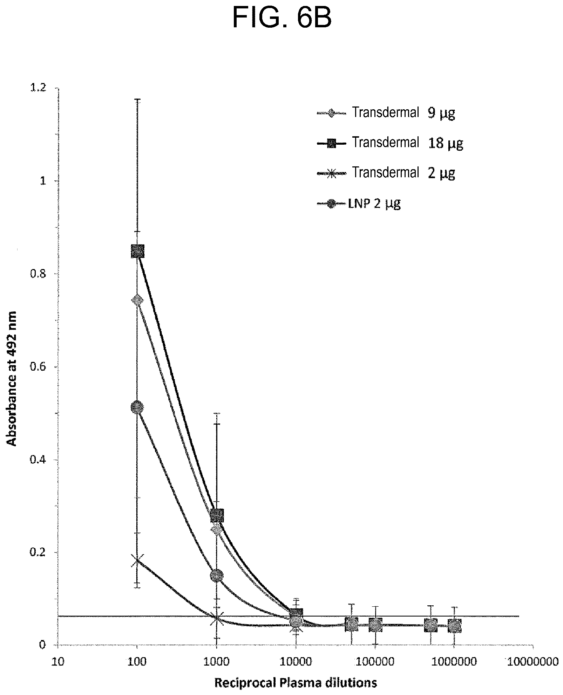

mΨU-modified OVA mRNA was administered at 2 µg (1 µg each to the right and left), 9 µg (4.5 µg each to the right and left), or 18 µg (9 µg each to the right and left) per mouse by the transdermal administration of the present invention. As a control, LNP containing 2 µg of the same mRNA as above was intramuscularly injected. Then, OVA-specific IgG in the mice was measured. The results were as shown in Figure 6B. As shown in Figure 6B, the transdermal administration of the present invention was found to induce antigen-specific IgG at a level equivalent to or more than that of the intramuscular injection of LNP.

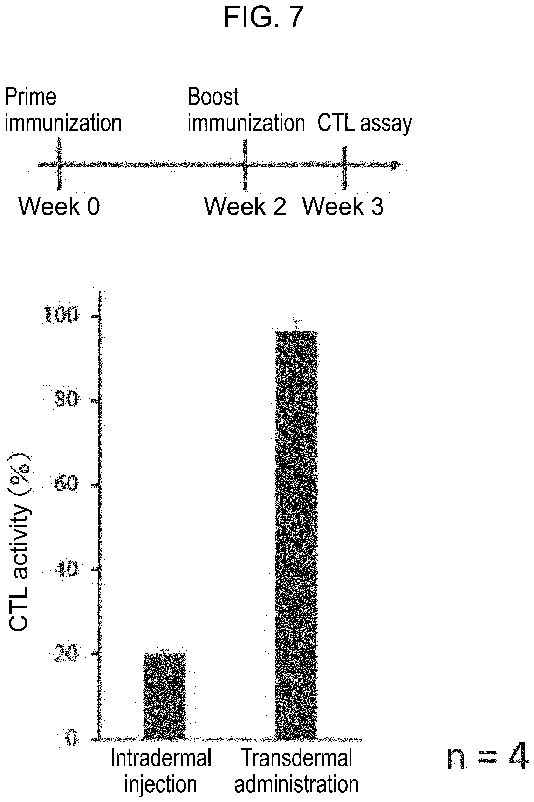

The induction of cytotoxic T cells was confirmed. According to the schedule as shown in Figure 7, 4.5 µg of unmodified OVA mRNA and 4.5 µg of unmodified OVA mRNA per mouse were transdermally administered to the right and left ventral parts, respectively, using Actranza(TM) Lab (transdermal administration group) or intradermally injected thereto (intradermal administration group). On week 3, splenocytes were recovered and treated with an OVA epitope, and the number of cells was confirmed in a flow cytometer. As a result, as shown in Figure 7, CTL activity (%) was significantly increased for the transdermal administration compared with intradermal injection.

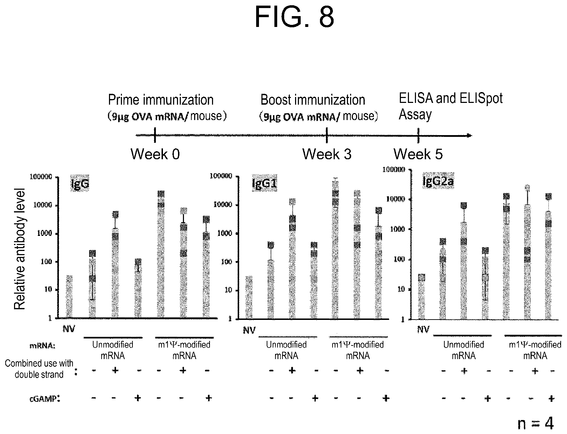

An effect of combined use with an adjuvant was further confirmed. The adjuvant used was double-stranded RNA (RIG-I ligand) or cGAMP (STING activator). Unmodified naked mRNA or m1Ψ-modified mRNA encoding ovalbumin was administered as mRNA to mice (n = 4). Plasma was recovered, and IgG, IgG1, and IgG2a levels in the plasma were measured by ELISA. The results were as shown in Figure 8. As shown in Figure 8, the induction of IgG, IgG1, and IgG2a was found in all the cases. An IgG2a/IgG1 ratio was determined, consequently revealing that the ratio was improved in all the adjuvant combined use groups. This suggests that the risk of antibody-dependent enhancement of infection may be reduced by combined use with the adjuvant. The double-stranded RNA was found to have an adjuvant effect in the case of using unmodified mRNA and however, found to have no adjuvant effect in the case of using m1Ψ mRNA.

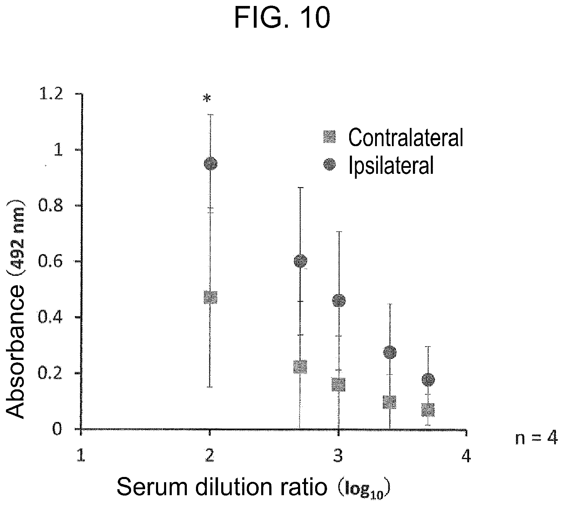

Next, 9 µg of the m1Ψ-modified mRNA encoding ovalbumin prepared as described above was administered to one ventral part in each mouse, and 3 weeks later, the same amount as above of the m1Ψ-modified mRNA was readministered to the ventral part on the same side as above (ipsilateral group, n = 4) or readministered to a ventral part on the opposite side (contralateral group, n = 4). Plasma was recovered from each of the groups, and an antibody titer was compared therebetween. As a result, as shown in Figure 10, markedly high antibody induction was found in the ipsilateral group compared with the contralateral group.

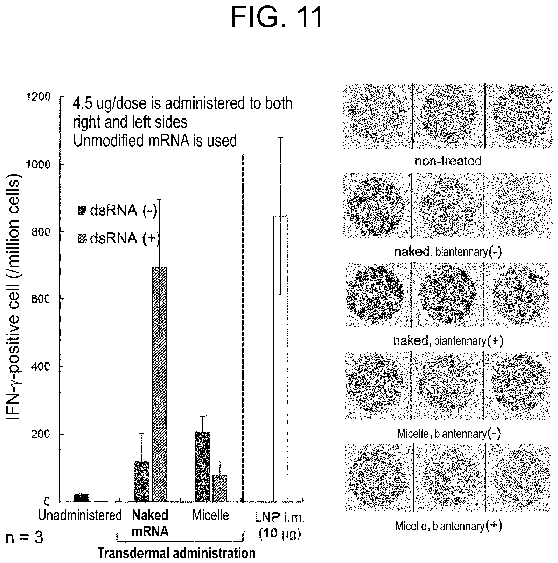

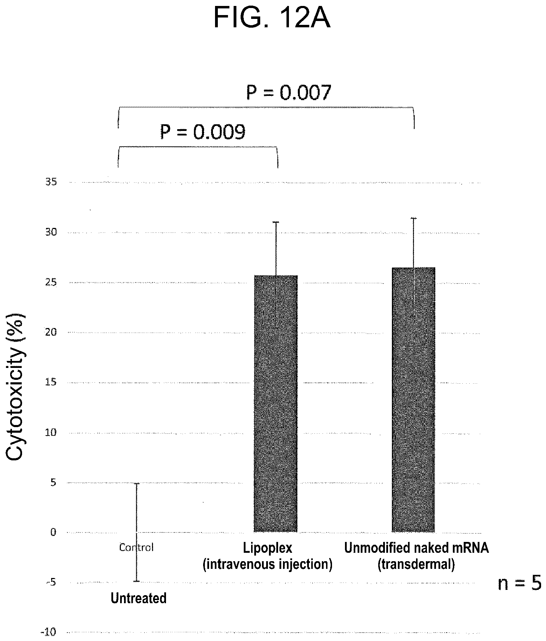

Unmodified naked mRNA encoding coronavirus helicase protein was prepared as described above. For this mRNA, groups supplemented with or without double-stranded RNA were provided in the same manner as above. As a control, a micelle group and a lipid nanoparticle (LNP) group were provided in which the mRNA was encapsulated. The mRNA-encapsulated micelle was obtained by mixing PEG-PAsp (DET) with the mRNA at an N/P ratio of 5 (e.g., see Kim H. J. et al., ACS Cent. Sci., 5, 1866-1875 (2019)). The naked mRNA and the micelle were transdermally administered using Actranza(TM) Lab. The LNP was administered by intramuscular injection. The results were as shown in Figure 11. As shown in Figure 11, the significant induction of INF-γ-positive cells was confirmed in the transdermal administration group of the naked mRNA and the transdermal administration group of the micelle. Particularly, the group given the double-stranded RNA (sdRNA) and the naked mRNA concurrently exhibited a very high effect of inducing specific immunity. The effect of inducing specific immunity in the group given the double-stranded RNA (sdRNA) and the naked mRNA concurrently was strong and comparable to the inducing effect of the LNP analogous to LNP from BioNTech currently used as a coronavirus vaccine (see Figure 12A).

The transdermal administration of naked mRNA according to the present invention can induce lymphoid follicle in the skin (particularly, the dermis). This lymphoid follicle is suggested to contribute to the induction of antigen-specific immunity by the transdermal administration of the naked mRNA.

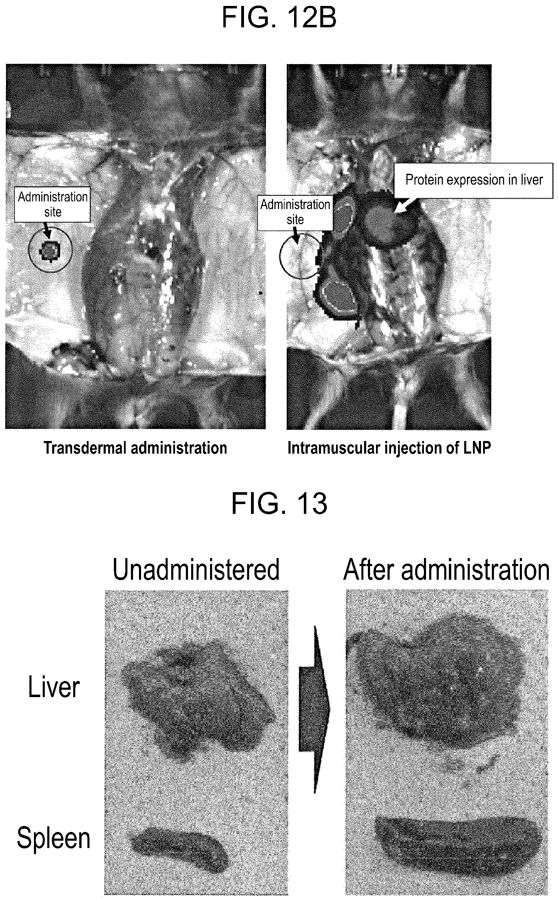

Various problems to lipid nanoparticle (LNP)-type mRNA vaccines are known. For example, such a vaccine, when intramuscularly injected, mostly migrates to the liver where mRNA is expressed (Figure 12B; and J. Control. Release, 217: 345-351, 2015). For example, the liver or the spleen swells after LNP administration (Figure 13). Thus, pH-responsive lipids have been reported to have strong immunogenicity (Immunity, 54 (12): 2877-2892, 2021), leading to toxicity. Such lipid nanoparticles, although confirmed to have effectiveness for nucleic acid delivery, present toxicity problems. Thus, expectations for dosage form design that avoids use of lipid nanoparticles are considered large. The transdermal administration of naked mRNA according to the present invention can be a possible solution to this problem. Although an experiment was carried out to transdermally administer unmodified naked mRNA by electric stimulation, the nucleic acid neither permeated the skin nor exhibited detectable expression in the skin by the electric stimulation.

Naked mRNA encoding coronavirus (Wuhan strain) spike protein was prepared as described above. This mRNA had m1Ψ modification in all uracil residues. This naked mRNA (dose: 5 µg, 10 µg, or 30 µg) was transdermally administered to each mouse (BALB/c and C58BL6/J) using Actranza(TM) Lab. The administration was performed twice at a 3-week interval. Blood was recovered from the mouse 2 weeks after the second administration, and plasma was obtained and analyzed for an antibody titer, for a neutralizing antibody, and for cellular immunity.

The antibody titer was measured using a spike protein-immobilized plate. Specifically, the spike protein (bicarbonate buffer solution (50 mM, pH = 9.6) containing 2 µg/mL of the protein) was added at 50 µL/well to Clear Flat-Bottom Immuno Nonsterile 96-Well Plates (Thermo Fisher Scientific Inc.) and incubated overnight at 4°C so that the spike protein was immobilized on the bottom of each well. The plate was washed three times with PBS containing 0.5% Tween 20 (PBS-T). 100 µL of a PBS-T solution (containing 1% BSA and 2.5 mM EDTA) was added to each well and incubated at room temperature for 1 hour. The plate was washed three times with PBS-T. 50 µL of an appropriately diluted plasma sample was added to each well and incubated overnight at 4°C. The plate was washed three times with PBS-T. 50 µL of a horseradish peroxidase (HRP)-labeled goat anti-mouse IgG antibody (1:8000) was added to each well and incubated at room temperature for 2 hours so that the labeled antibody was bound to a plasma antibody bound to the immobilized spike protein. After washing, HRP substrate was added at 100 µL/well and incubated at room temperature for 30 minutes. The reaction was terminated with 2 M sulfuric acid, and absorbance at 492 nm was observed with a plate reader (Tecan) to measure the amount of the labeled antibody bound to the plasma antibody bound to the spike protein.

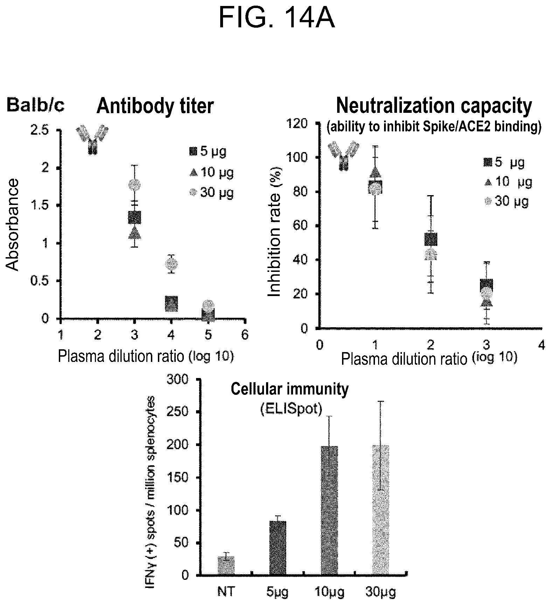

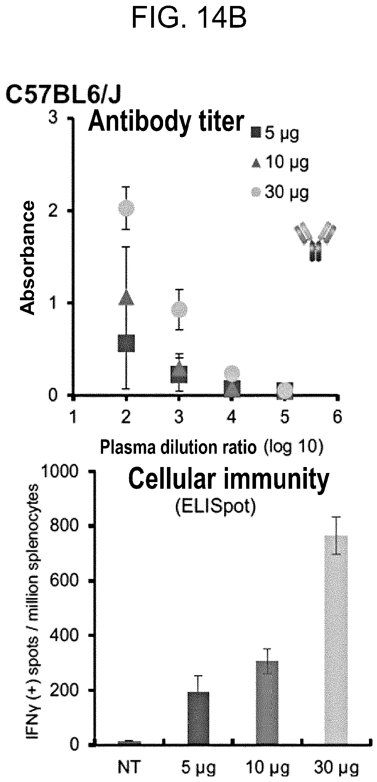

The results were as shown in Figures 14A and 14B. As shown in Figure 14A, the BALB/c mouse group given the mRNA encoding the spike protein by the transdermal administration method of the present invention exhibited an antibody titer of 10,000 or more.

Next, the neutralization capacity (specifically, neutralization capacity for the binding between the spike protein and ACE2) of the antibody in the obtained plasma was evaluated. The neutralization capacity for spike protein RBD and ACE2 was quantified using SARS-CoV-2 Spike RBD-ACE2 Blocking Antibody Detection ELISA Kit (manufactured by Cell Signaling Technologies, Inc.). As a result, as shown in Figure 14A, the presence of an antibody having neutralization capacity was certainly found in the obtained plasma.

Splenocytes of the mice given the mRNA were further recovered and evaluated for the activation of antigen-specific cellular immunity. The recovered splenocytes were inoculated at a density of 2.5 × 10⁵ cell/well to a 96-well plate of Mouse Anti-IFNy ELISpot PLUS kits (Mabtech AB). 24 hours later, the plate was treated in accordance with the instruction of the kit and observed with ELISpot plate reader (AID GmbH, Germany). In ELISpot assay, antigen-dependent increase in the number of IFNy-positive spots by the transdermal administration was found, indicating that antigen-specific cellular immunity was activated.

When the same experiment as above was carried out on C58BL6/J mice, similar results were shown. Specifically, as shown in Figure 14B, a high antibody titer and the activation of cellular immunity were confirmed when unmodified naked mRNA encoding the spike protein was transdermally administered by the method of the present invention.

As mentioned above, the transdermal administration by the method of the present invention was capable of administering naked mRNA and was able to induce the production of an antibody against the protein encoded by the thereby administered mRNA, and cellular immunity.

The amounts of inflammatory cytokines produced in the liver and the spleen were quantified after transdermal administration by the method of the present invention. LNP was obtained by mixing D-Lin-MC3-DMA, DSPC, cholesterol, and PEG2000-DMG (molar ratio 50:10:38.5:1.5, ethanol solvent) with mRNA (citrate buffer of pH 3) (N/P = 5) at a flow rate of 12 mL/min in a microchannel (Precision NanoSystems Inc., Vancouver, BC, Canada). Then, the buffer was replaced with PBS. OVA mRNA (m1Ψ) in a form encapsulated in the LNP was intradermally administered, or the mRNA alone was transdermally administered using Actranza Lab. 4 hours later, the liver and the spleen were excised. Total RNA was extracted using RNeasy mini kit (Qiagen N.V.) and reverse-transcribed using ReverTraAce with gDNA remover kit (Toyobo Co., Ltd.). Quantitative PCR was performed using 7500 fast real time PCR (Applied Biosystems), FastStart Universal SYBR Green Master kit, and primers forward: GAACCAGATCACCAAGCCCA, reverse: GTACAGCTCCTTCACGCACT. In the quantitative PCR, Taqman Gene Expression Assays (IL-6 (Mm00446190_m1), IFN-β (Mm00439552_s1), β actin (Mm00607939)) were used.

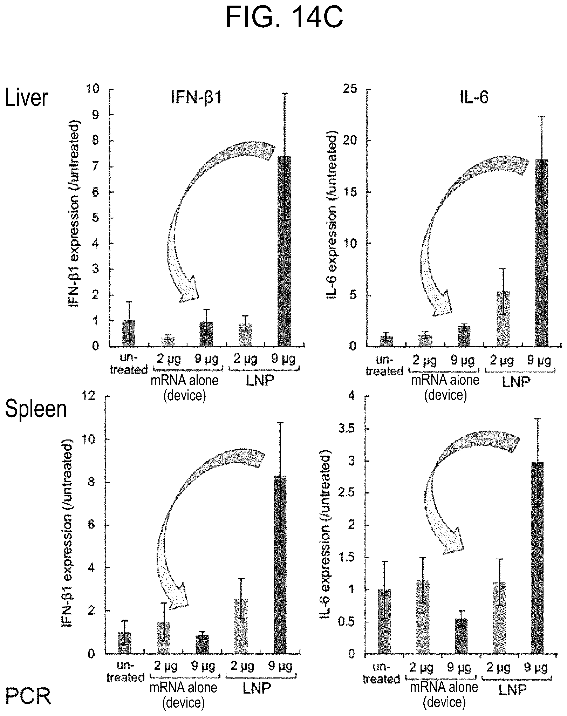

The results were as shown in Figure 14C. As shown in Figure 14C, the group transdermally given the mRNA produced few inflammatory cytokines in both the liver and the spleen, whereas the LNP administration group induced a high level of inflammatory cytokines in both the liver and the spleen.

mRNA was transdermally administered to the back of cynomolgus monkeys three times at 3-week intervals using Actranza(TM) Lab, and blood was collected over time and evaluated for an antibody titer and neutralization capacity. The antibody titer was evaluated using HRP-conjugated Goat Anti-Monkey IgG H&L (1 :8000) instead of goat anti-mouse IgG (1 :8000). As a result, as shown in Figure 14D, the production of an antibody against the spike protein was also found in the plasma of the cynomolgus monkeys. The obtained antibody had neutralization capacity. Figure 2B shows photographs of an administration site after mRNA administration in the monkeys.

This suggested that the transdermal administration of the present invention requires administration using a device having sufficient ejection power for allowing an mRNA solution to permeate the skin by a jet spray. Particularly, it is considered that naked mRNA, even when transdermally administered by ejection, can be delivered into cells without damage on the mRNA. It is also evidently important to allow the mRNA solution to widely permeate the skin by a jet spray. The jet spray can be performed at a plurality of divided stages. mRNA was able to be administered deeply (particularly, to the dermis) and widely to the skin by the first stage of spraying for improvement in skin permeability and the subsequent second stage of spraying of the mRNA solution. The mRNA ejected by the jet sprays is considered to permeate tissues in a wide region and at a high depth and be also introduced into cells. Based on the conventional knowledge, the administration of mRNA in a naked state to a living body has been avoided because the mRNA is rapidly degraded by RNase abundantly present *in vivo.* By contrast, the transdermal administration method of the present invention can stably deliver mRNA into cells without degradation or disruption problems, and can deliver a translation product encoded by the mRNA to tissues such as the epidermis or the dermis. In the case of intradermal injection, an administered component is systemically spread without remaining at a local site, whereas a jet spray prevents such an event. Hence, the spray administration of a component by a jet spray seems to be effective for reducing undesirable side effects. Particularly, for the activation of immunity, this method is capable of significantly inducing an antibody or immunocytes in an antigen-specific manner even if an active ingredient remains intradermally. These results suggest that the transdermal administration of a component by a jet spray is suitable for vaccination.

Jet Injector from CureVac N.V. exhibits approximately 1.5-fold to 10-fold improvement at most in expression level with respect to administration by injection and rarely has a vaccine effect. The method of the present invention enabled mRNA to widely and deeply permeate the dermis, improved expression by more than 100 times with respect to intradermal injection, and also improved a vaccine effect (antibody production) by 100 or more times. It was found desirable for effectively delivering mRNA into immunocytes (particularly, dendritic cells, particularly, dendritic cells of the dermis) scattered in the skin that an active ingredient can be delivered to the dermis and can be administered to a wide area. Administration may be performed a plurality of times in order to expand the area where a drug solution permeates the skin. Thus, a vaccine effect can be expected by administration once or more and the permeation of an active ingredient in a wide region of the skin. The present invention proposes an administration method suitable for vaccines, also including such an administration method.

Existing mRNA vaccines are intramuscularly injected in the form of mRNA encapsulated in lipid nanoparticles. However, many vaccines are refrigerable at 10°C or lower, whereas the mRNA vaccines inevitably require cryopreservation at -20°C or -80°C and are considered to reduce their effects under insufficient temperature control. It has also been pointed out that lipid components contained in the mRNA vaccines may cause allergic response. By contrast, the transdermal administration method of the present invention can administer mRNA in a naked form and can thereby induce antigen-specific immunity in the body. This suggests that safe administration with very low toxicity is achieved when naked mRNA is used. The method of the present invention can also be beneficial from the viewpoint of reducing production cost thereof because there is room for simplifying components.

### Description of Reference Sings in Drawings

10: RNA formulation, 11: pressurization port, 11a: plunger, 11b: plunger rubber, 12: spray port, 13: composition comprising RNA, 14: container of the RNA formulation, 20: gas generation unit, 21: container of the gas generation unit, 23: gas-generating agent, 24: ignition plug, 30: administration device before connected to the gas generation unit, 31: body, 32: handle part, 33: plug, 34: switch, 35: voltage generation unit, 36: conductive wire, 37: conductive wire, 38: holder unit for the fixation of the gas generation unit, 50: administration device linked to the gas generation unit, 100: RNA formulation linked to the administration device having the gas generation unit

### [Sequence Listing]

Sequence Number (ID): 1
   Length: 24
   Molecule Type: RNA
   Features Location/Qualifiers:
      - misc_feature, 1..24
         > note, RNA 1 for double strand RNA
      - source, 1..24
         > mol_type, other RNA
         > organism, synthetic construct
   Residues:
      ggtgtgtgtg tgtgtgtgtg tgtg 24
Sequence Number (ID): 2
   Length: 37
   Molecule Type: DNA
   Features Location/Qualifiers:
      - misc_feature, 1..37
         > note, DNA template
      - source, 1..37
         > mol_type, other DNA
         > organism, synthetic construct
   Residues:
      acgactcact ataggtgtgt gtgtgtgtgt gtgtgtg 37
Sequence Number (ID): 3
   Length: 43
   Molecule Type: RNA
   Features Location/Qualifiers:
      - misc_feature, 1..43
         > note, RNA 2 for double strand RNA
      - source, 1..43
         > mol_type, other RNA
         > organism, synthetic construct
   Residues:
      ggttcaggat gtcccgcttc acacacacac acacacacac acc 43
Sequence Number (ID): 4
   Length: 43
   Molecule Type: RNA
   Features Location/Qualifiers:
      - misc_feature, 1..43
         > note, RNA 3 for double strand RNA
      - source, 1..43
         > mol_type, other RNA
         > organism, synthetic construct
   Residues:
      gggcaggatg gggtaccttc acacacacac acacacacac acc 43
Sequence Number (ID): 5
   Length: 43
   Molecule Type: RNA
   Features Location/Qualifiers:
      - misc_feature, 1..43
         > note, RNA 4 for double strand RNA
      - source, 1..43
         > mol_type, other RNA
         > organism, synthetic construct
   Residues:
      gtcctcgtcc ttgaaggatc acacacacac acacacacac acc 43
Sequence Number (ID): 6
   Length: 43
   Molecule Type: RNA
   Features Location/Qualifiers:
      - misc_feature, 1..43
         > note, RNA 5 for double strand RNA
      - source, 1..43
         > mol_type, other RNA
         > organism, synthetic construct
   Residues:
      gcttctcgaa gttgatgtac acacacacac acacacacac acc 43
Sequence Number (ID): 7
   Length: 43
   Molecule Type: RNA
   Features Location/Qualifiers:
      - misc_feature, 1..43
         > note, RNA 6 for double strand RNA
      - source, 1..43
         > mol_type, other RNA
         > organism, synthetic construct
   Residues:
      ggcgatgtgc ttgatgcttc acacacacac acacacacac acc 43

## Claims

1. A composition comprising
a nucleic acid, the nucleic acid comprising RNA, wherein
the composition is administered by an administration method, the administration method comprising spraying the composition onto the surface of a target tissue, whereby the composition is delivered into the target tissue by penetrating the surface of the target tissue.

2. The composition according to claim 1, wherein the pressurization is caused by the generation of a gas from a gas-generating agent.

3. The composition according to claim 1 or 2, wherein the spraying is performed so as to introduce mRNA to an area of 70 mm² or larger.

4. The composition according to any one of claims 1 to 3, wherein the spraying is performed so as to introduce mRNA to an area of 200 mm² or larger.

5. The composition according to any one of claims 1 to 4, wherein the nucleic acid comprises naked mRNA.

6. The composition according to any one of claims 1 to 5, wherein the composition further comprises an adjuvant.

7. The composition according to claim 6, wherein the adjuvant comprises an RIG-I ligand or a STING activator.

8. The composition according to any one of claims 1 to 7, wherein at least some or all of the nucleic acids are contained in lipid nanoparticles or contained in polyion complexes.

9. The composition according to any one of claims 1 to 8,
(i) for use in delivering the nucleic acid to the epidermis or the dermis of a subject,
(ii) for use in expressing the nucleic acid in the epidermis or the dermis of a subject,
(iii) for use in inducing the formation of lymphoid follicle in a subcutaneous tissue of a subject, and/or
(iv) for use in inducing specific immunity against a translation product of the nucleic acid in a subject.

10. The composition according to any one of claims 1 to 9,
(iv-1) for use in inducing an antibody against a translation product of the nucleic acid in a subject.

11. The composition according to any one of claims 1 to 10,
(iv-2) for use in inducing T cell immunity against a translation product of the nucleic acid in a subject.

12. A method for administering a nucleic acid to a subject in need thereof, the nucleic acid comprising RNA, the method comprising spraying the composition to the surface of a target tissue of the subject, whereby the composition is sprayed toward the target tissue and delivered into the target tissue by penetrating the surface of the target tissue.

13. A method for inducing antigen-specific immunity in a subject in need thereof, the method comprising spraying a composition comprising mRNA encoding the antigen to the skin surface of the subject, whereby the composition is sprayed toward the target tissue and delivered into the target tissue by penetrating the surface of the target tissue.

14. The method according to claim 12 or 13, wherein the RNA consists of naked mRNA.

15. An administration device comprising an RNA formulation, comprising
(α) the administration device having a gas generation unit comprising at least a gas-generating agent, the gas generation unit having a driving unit for driving a plunger by a pressure ascribable to a gas generated from the gas-generating agent in the gas generation unit, and
(β) the RNA formulation having a composition comprising RNA and a container where the composition is stored, the container having a pressurization port for pressurization and a spray port for spraying, wherein
the plunger is air-tightly and liquid-tightly inserted to the pressurization port of the RNA formulation, driven by the driving unit through gas generation in the gas generation unit, and thereby pushed into the container, whereby the composition comprising RNA is capable of being ejected from the spray port.

16. An RNA formulation comprising
a composition comprising RNA and a first container where the composition is stored,
the container having a pressurization port for pressurization and a spray port for spraying, wherein a plunger is inserted to the pressurization port and is capable of being pushed into the first container, and when the plunger is pushed into the first container from the pressurization port (i.e., pressurization is caused), the composition comprising RNA is sprayed from the spray port, whereby at least the RNA penetrates tissue surface and permeates the inside of the tissue.

17. The RNA formulation according to claim 16, further comprising
a gas generation unit (actuator), the gas generation unit having a gas generation unit (actuator) having a second container comprising at least a gas-generating agent, the gas generation unit (actuator) having a driving unit for pushing the plunger, wherein the plunger is capable of being pushed into the first container via the driving unit by a gas generated from the gas-generating agent, and when the plunger is pushed into the first container from the pressurization port (i.e., pressurization is caused), the composition comprising RNA is sprayed from the spray port, whereby at least the RNA penetrates tissue surface and permeates the inside of the tissue.

18. The RNA formulation according to claim 17, further having
a body part of an administration device,
the body part having a handle part, a switch, a voltage generation unit electrically connected with the switch, and a plug electrically connected with the voltage generation unit, and having a holder unit that permits fixation of the gas generation unit (actuator), wherein the gas generation unit is fixed to the holder unit, and the plug of the body is electrically connected with an ignition plug of the gas generation unit (actuator), whereby the RNA formulation is configured such that a voltage is generated from the voltage generation unit by switch-on so that the ignition plug is set on fire and a gas is generated from the gas-generating agent in the second container of the gas generation unit.
